# FASCICULE DE BREVET EUROPEEN

(11) **EP 3 377 623 B1**
(45) Date de publication et mention de la délivrance du brevet: **20.10.2021**
(21) Numéro de dépôt: 16794324.0
(22) Date de dépôt: 10.11.2016
(51) Int. Cl.: C12N 15/10, C07H 1/08, C07H 21/02, A61K 8/60, A61K 8/97

(54) **PROCEDE D'OBTENTION D'UN EXTRAIT AQUEUX ENRICHI EN PETITS ARN A PARTIR D'UNE MATIERE VEGETALE ET EXTRAITS ISSUS DU PROCEDE**
VERFAHREN ZUR GEWINNUNG EINES MIT SMALL-RNA AUS PFLANZENMATERIAL ANGEREICHERTEN WÄSSRIGEN EXTRAKTS UND AUS DIESEM VERFAHREN GEWONNENE EXTRAKTE
PROCESS FOR OBTAINING AN AQUEOUS EXTRACT ENRICHED WITH SMALL RNAS FROM A PLANT MATERIAL AND EXTRACTS RESULTING FROM THE PROCESS

(30) Priorité: 17.11.2015 FR 1502361
(43) Date de publication de la demande: 26.09.2018
(73) Titulaire: ISP Investments LLC, Wilmington, DE 19805 (US)
(72) Inventeur: PORTOLAN, Frédérique, 06560 Valbonne (FR); OGER, Elodie, 06220 Vallauris (FR); LEQUOY, Valérie, 06560 Valbonne (FR)
(74) Mandataire: Miquel, Corinne
(86) Numéro de dépôt international: PCT/EP2016/077231
(87) Numéro de publication internationale: WO 2017/084958

(56) Documents cités:
- EP-A2- 1 723 958
- WO-A1-02/057289
- WO-A2-03/101376
- WO-A2-2004/046305
- WO-A2-2005/034648
- CN-A- 101 601 635
- DE-A1- 19 820 629
- FR-A1- 2 831 168
- FR-A1- 2 912 313
- FR-A1- 2 940 112
- Pingjing Zhang ET AL: "Use of small RNA as antiaging cosmeceuticals", Journal of cosmetic science, 1 novembre 2013 (2013-11-01), pages 455-468, XP055282728, United States Extrait de l'Internet: URL:http://journal.scconline.org//pdf/cc20 13/cc064n06/p00455-p00468.pdf
- DEPAULO J J ET AL: "Extraction of double-stranded RNA from plant tissues without the use of organic solvents", PLANT DISEASE, THE AMERICAN PHYTOPATHOLOGICAL SOCIETY, US, vol. 79, no. 3, 1 janvier 1995 (1995-01-01), pages 246-248, XP009190702, ISSN: 0191-2917
- VANESSA DOS REIS FALCÃ GBP O ET AL: "RNA Isolation method for polysaccharide rich algae: agar producing Gracilaria tenuistipitata (Rhodophyta)", JOURNAL OF APPLIED PHYCOLOGY, KLUWER ACADEMIC PUBLISHERS, DO, vol. 20, no. 1, 20 juillet 2007 (2007-07-20), pages 9-12, XP019574461, ISSN: 1573-5176
- RONALD J SMERNIK ET AL: "Identification of RNA Hydrolysis Products in NaOH-EDTA extracts using 31P NMR Spectroscopy", INTERNET CITATION, 12 October 2015 (2015-10-12), pages 2746-2756, XP002775075, Retrieved from the Internet: URL:http://www.tandfonline.com/doi/full/10 .1080/00103624.2015.1093640 [retrieved on 2017-10-23]

## Description

L'invention concerne un procédé d'obtention d'un extrait aqueux enrichi en petits ARN (acides ribonucléiques) d'une longueur d'au maximum 150 nucléotides (nt) à partir d'une matière végétale, les extraits issus d'un tel procédé, ainsi que les compositions comprenant de tels extraits et leurs utilisations cosmétiques.

Les protocoles d'extraction classiques des acides ribonucléiques (ARN, ARN de petit poids moléculaire) réalisés en laboratoire, et donc à petite échelle, doivent se dérouler sous hotte chimique car ces procédés impliquent l'utilisation de solvants tels que le phénol et le chloroforme qui sont toxiques et ne sont pas considérés comme des solvants cosmétiques (Zumbo, P. 2014 "Phenol-chloroform Extraction", 2014). Ces solvants sont ajoutés à une phase aqueuse de cellules lysées ou de tissus végétaux ou animaux broyés très finement, soit directement sur le broyat réalisé en présence d'azote liquide. La solution ainsi obtenue est centrifugée à haute vitesse de l'ordre de 10000 g à 4°C, afin de créer deux phases bien distinctes, une phase organique contenant les protéines et une phase aqueuse contenant les ARN totaux, la phase intermédiaire contenant l'ADN. La phase aqueuse doit alors être récupérée avec soin, afin de ne pas prélever par inadvertance de la phase intermédiaire et organique. Une étape de précipitation des ARN est réalisée à l'isopropanol. Le culot d'ARN totaux est ensuite lavé avec de l'éthanol. Puis, les ARN totaux sont resolubilisés dans de l'eau et doivent être conservés à très basse température (-20°C et mieux encore -80°C).

Afin d'obtenir uniquement les ARN de petit poids moléculaire, il est possible d'utiliser des kits contenant des colonnes de purification comme le kit de chez Sigma, mirPremier™ microRNA Isolation Kit. Ces kits ne peuvent être utilisés qu'à une échelle de test en laboratoire car les volumes considérés sont de l'ordre du mL et ne peuvent donc pas être adaptés à un développement de produit à grande échelle.

De plus, tous ces protocoles d'extraction et purification ne vont permettre d'obtenir que la fraction d'acide nucléique purifiée. Cette fraction d'acide nucléique ARN ou ADN ou petits ARN sera dépourvue de tout autre molécule d'intérêt telle que les métabolites secondaires, vitamines, sucres, peptides, etc. qui peuvent avoir des effets bénéfiques pour la peau et présentent donc un intérêt cosmétique.

Par ailleurs, on connait par exemple le document WO8403835 qui décrit un procédé d'obtention d'un extrait aqueux d'embryons végétaux enrichi en ADN pur. Un tel procédé met en œuvre notamment des embryons de blé et/ou de soja broyés dans une solution d'extraction tamponnée à pH 9,5 renfermant du saccharose, de l'EDTA (0,05 M) et du chlorure de sodium. Après filtration, le résidu est remis en suspension dans une solution tris salin tamponnée à pH 7,4 additionnée de détergent anionique sous agitation à température ambiante, puis ajout de perchlorate de sodium sous agitation à 0°C, et ajout de chloroforme et d'octanol avant centrifugation à basse température (4°C) et très haute vitesse (25000 g). Une phase aqueuse renfermant essentiellement de l'ADN avec une petite quantité d'ARN et d'acides aminés est récupérée, sur laquelle on peut faire avantageusement agir une RNAse.

Ce document divulgue donc l'obtention d'un extrait aqueux d'embryons de végétaux enrichi spécifiquement en ADN en utilisant un grand nombre de traitement, dont des traitements par un détergent anionique et différents solvants dont le chloroforme et l'octanol qui sont susceptibles de laisser des traces toxiques dans les produits obtenus et ne peuvent ainsi être utilisés en cosmétique.

On connait également le document FR2831168 qui décrit un procédé d'obtention d'un extrait riche en acides nucléiques (ADN et/ou ARN) à partir d'une matière végétale, notamment d'embryons végétaux ou de graines riches en ADN ou ARN. Le procédé consiste à extraire en milieu aqueux la matière végétale en présence d'enzymes cellulolytiques à un pH initial de 9 à 13, le pH évoluant vers la neutralité (pH optimum pour l'action de ces enzymes) en quelques minutes, puis séparer la matière végétale pour récupérer un extrait aqueux, et enfin traiter l'extrait par une protéase et séparer les insolubles pour récupérer un extrait aqueux purifié. Le produit lyophilisé ainsi obtenu peut contenir en particulier de 0,1 à 1% en poids d'ADN, de 0,2 à 1,5% en poids d'ARN ainsi que des glucides, protéines, minéraux, vitamine B et lipides.

D'après les données décrites dans ce document, le produit lyophilisé obtenu semble donc contenir notamment de 1 à 10 mg/L d'ADN et de 10 à 75 mg/L d'ARN.

Considérant ce qui précède, un problème que se propose de résoudre l'invention est de développer un procédé d'obtention d'un extrait aqueux enrichi spécifiquement en petits ARN et ne comportant pas d'ADN, à partir d'une matière végétale, qui soit facile et peu coûteux à mettre en œuvre à l'échelle industrielle, ne nécessitant pas obligatoirement l'utilisation d'enzymes cellulolytiques (cellulase, hemicellulase) ou de DNAse, offrant de bons rendements en petits ARN, et ne présentant pas les inconvénients des procédés de l'art antérieur cité, comme par exemple l'utilisation de détergent et de solvants potentiellement toxiques.

L'extrait ainsi obtenu peut alors être utilisé directement en cosmétique.

L'invention a donc pour premier objet un procédé d'obtention d'un extrait aqueux enrichi en petits ARN d'une longueur d'au maximum 150 nucléotides à partir d'une matière végétale comprenant les étapes suivantes:
a) on met en présence la matière végétale avec de l'eau ;
b) on ajoute de l'acide éthylène diamine tétraacétique (EDTA) tétrasodique dans le mélange obtenu en a), le pH du mélange étant compris entre 10,5 et 11 ;
c) on ajuste ensuite le pH du mélange obtenu en b) à une valeur comprise entre 6 et 8 ;
d) on purifie le mélange obtenu en c) de manière à éliminer la matière végétale et récupérer un extrait brut aqueux ; et
e) on réalise au moins une filtration de l'extrait brut aqueux pour obtenir l'extrait aqueux enrichi en petits ARN d'une longueur d'au maximum 150 nucléotides dont le pH est contrôlé et si nécessaire réajusté à une valeur comprise entre 6 et 8, préférentiellement entre 6 et 6,5.

De plus, l'invention a pour deuxième objet un extrait aqueux de matière végétale, enrichi en petits ARN d'une longueur d'au maximum 150 nucléotides obtenu par le procédé selon l'invention, caractérisé en ce qu'il comprend en poids du poids total de l'extrait, de 5 à 60 g/kg d'extrait sec et 10 à 1000 mg/kg de petits ARN d'une longueur d'au maximum 150 nucléotides et ne comprend pas d'ADN.

L'invention a pour troisième objet une composition comprenant, en tant qu'agent actif anti-âge, une quantité efficace d'un extrait selon l'invention, et un milieu physiologiquement acceptable.

L'invention a pour quatrième objet l'utilisation cosmétique d'une composition selon l'invention pour lutter contre les signes du vieillissement cutané.

Enfin, l'invention a pour cinquième objet l'utilisation cosmétique d'une composition selon l'invention pour améliorer l'hydratation de la peau.

L'invention et les avantages qui en découlent seront mieux compris à la lecture de la description et des modes de réalisation non limitatifs qui suivent, rédigée au regard des figures annexées dans lesquelles :
- la Figure 1A représente une électrophorèse sur gel d'agarose à 2% mettant en évidence l'effet du pH sur l'extraction d'ARN de petit poids moléculaire dans des extraits selon l'exemple 2 (germes de riz) de l'invention ;
- la Figure 1B représente une électrophorèse sur gel d'agarose à 2% mettant en évidence l'effet d'une hydrolyse enzymatique sur l'extraction d'ARN de petit poids moléculaire dans des extraits selon l'exemple 2 (germes de riz) de l'invention ;
- la Figure 2 représente la quantification au Bioanalyseur® des ARN de petit poids moléculaire d'un extrait selon l'exemple 3 (lentilles) de l'invention ;
- la Figure 3 représente la quantification au Bioanalyseur® des ARN de petit poids moléculaire de l'extrait selon l'exemple 4 (lentilles) de l'invention avant et après traitement à la DNAse ;
- la Figure 4A représente l'effet de différents extraits de baobab (*Adansiona digitata*) selon les exemples 7, 8 et 9, sur l'expression de l'hyaluronane synthase 2 (HAS2) sur des fibroblastes humains de passage 8 (P8) ; et
- la Figure 4B représente l'effet de différents extraits de baobab (*Adansiona digitata*) selon les exemples 7, 8 et 9, sur l'expression de l'hyaluronane synthase 2 (HAS2) sur des fibroblastes humains de passage 32 (P32).

Dans cette description, sauf indication contraire, il est entendu que, lorsqu'un intervalle est donné, il inclut les bornes supérieure et inférieure dudit intervalle.

L'invention concerne un procédé mis en œuvre pour l'obtention d'un extrait aqueux enrichi en petits ARN d'une longueur d'au maximum 150 nucléotides à partir d'une matière végétale. Il est à noter que le procédé de l'invention peut être précédé par tout type d'étape préliminaire d'extraction connues de l'homme du métier (hydrolyse chimique, enzymatique...) permettant par exemple d'éliminer préalablement certaines fractions de plante qui pourraient nuire au bon déroulement du procédé de l'invention.

Par « petits ARN » ou « ARN de petit poids moléculaire », on entend des ARN (acides ribonucléiques) non codants de petit poids moléculaire, d'une longueur d'au maximum 150 nucléotides, tels que tous types de petits ARN non messager, simple et/ou double brins, par exemple les micro-ARN, les ARN interférents, les introns, les petits ARN nucléaires ou encore tout fragment d'ARN.

Une matière végétale est de manière générale une plante, organisme vivant faisant partie du règne végétal caractérisé par une très faible motilité, qui se nourrit de substances minérales et absorbe du gaz carbonique, et inclut les plantes dont le cycle de vie se déroule généralement en milieu aquatique telles que les algues.

Avantageusement, *pour la mise en oeuvre du procédé,* la matière végétale selon l'invention est une plante entière ou préférentiellement une partie de plante (*fruit, feuilles, racines, bulbes, germes, graines...*), *qui peuvent être utilisées sous forme fraiches, sèches, germées, entières, réduites en poudre, congelées, mais aussi sous forme d'un* résidu de plante obtenu après transformation comme *les tourteaux, ou les drèches. Pour* se *faire toutes les parties des espèces de plantes citées peuvent être utilisées pour obtenir un extrait* extrait aqueux enrichi en petits ARN d'une longueur d'au maximum 150 nucléotides. Ainsi les exemples concernant certaines parties de la plante sont donnés à titre illustratif et non limitatif.

Peuvent être considéré avantageusement comme matière végétale de départ pour obtenir un extrait aqueux enrichi en ARN de petit poids moléculaire n'importe quel fruit ou partie de fruit. On peut par exemple citer les fruits entiers, en morceaux, sous forme de poudre, frais ou congelé, sous forme de drèche. On peut citer les fruits de la famille des *Passifloraceae,* mais également des fruits d'autres familles, tels ceux de la famille des *Malvaceae* comme *Hibiscus esculentus* (le gombo). On peut également citer des fruits de la famille des *Punicaceae* comme *Punica granatum* (la grenade), ceux de la famille des *Actinidiaceae* comme *Actinidia chinensis (le kiwi),* de la famille des *Myrtaceae* comme *Psidum guajava (la goyave),* les fruits de la famille des *Clusiaceae* comme *Garcinia cambodgia* (le tamarinier de Malabar), de la famille des *Caricaceae* comme *Carica papaya (la papaye).* Mais encore les fruits appartenant à la famille des *Malphigiaceae* comme *Malphigia glabra* (l'acérola), les fruits sous forme de baies comme ceux de la famille des *Ericaceae* comme *Vaccinium myrtillus* (la myrtille) et de la famille des *Grossulariaceae* comme *Ribes nigrum (le cassis).* Ou encore tout fruit de la famille des *Solanaceae,* tel que *Lycium barbarum* (les baies de goji).

Un extrait aqueux enrichi en ARN de petit poids moléculaire peut également être obtenu à partir de n'importe quelle fleur sous forme entière, en poudre, fraiche ou congelée, par exemple de la famille *des Rosaceae* comme les roses ou de la famille des *Rutaceae* comme *Citrus aurantium (fleur de bigaradier).* On peut encore citer l'utilisation des fleurs d'autres familles telles que les fleurs de la famille des *Oleaceae* comme *Jasminum officinalis* (le jasmin) ou encore les fleurs de la famille des *Asteraceae comme Centaurea cyanus (le bleuet).*

Les graines peuvent également être utilisées comme matière végétale de départ pour obtenir un extrait aqueux enrichi en ARN de petit poids moléculaire. N'importe quelle graine, entière sous forme de poudre, germées ou non germées. A titre indicatif et non restrictif les graines peuvent être issues de plante de la famille des *Chenopodiaceae comme Quinoa chenopodium,* de la famille des légumineuses ou *Fabaceae* comme *Lens esculenta* (la lentille) ou encore appartenant à la famille des *Poaceae* comme *Oryza sativa* (le riz). Ou encore, de la famille des *Cucurbitaceae* comme la *Cucurbita pepo* (la courge), ou encore des *Lamiaceae.* Egalement utilisables pour l'invention, les petites graines ou pépins de la famille des *Rutaceae,* ou *Rosaceae* ou tout autre espèces produisant des fruits contenant des pépins ou noyaux. Les parties souterraines d'une plante telles que les racines, ou les rhizomes, les bulbes peuvent être également considérés comme matière végétale de départ pour obtenir un extrait aqueux enrichi en ARN de petit poids moléculaire. Ces parties sous-terraines, peuvent se présenter sous forme entière, de poudre, fraiches, séchées ou congelées. A titre indicatif et non restrictif les racines ou bulbes ou rhizomes, provenir de la famille des *Liliaceae comme Lilium candidum ou Lilium tigrinum,* mais peuvent également être considérée pour l'invention toutes espèces de plante de la famille des *Iridaceae, des Amaryllidaceae, des Alliaceae.*

Peuvent être également considéré comme matière végétale de départ pour obtenir un extrait aqueux enrichi en ARN de petit poids moléculaire n'importe quel type de feuille sous forme entière, de poudre, fraiche ou congelée, on peut citer les plantes de la famille des *Araliaceae* ou *Dioscoreaceae* ou tout autre type de famille de plante possédant un système foliaire développée.

Les plantes utilisées dans les exemples sont préférentiellement choisies parmi la famille des *Poaceae* (anciennement graminées), *Fabaceae* appelée couramment légumineuses, *Malvaceae, Bombacaceae, Cucurbitaceae, Chenopodiaceae* ou pseudo céréale, *Rosaceae, Rutaceae, liliaceae, Passifloraceae.*

A titre d'exemple illustratif non limitatif, la matière végétale est choisie parmi les espèces *Hibiscus esculentus* (gombo), *Adansonia digitata* (baobab), *Chenopodium quinoa* (quinoa), *Lens esculenta* (lentille), *Oryza sativa* (riz), *Cucurbita pepo* (courge), *Rosa centifolia (rose), Citrus aurantium (oranger amère), Lilium candidum (lis), Lilium tigrinum (lis tigré), Passiflora alata (grenadille).*

Dans une première étape a) du procédé selon l'invention, on met en présence la matière végétale avec de l'eau, préférentiellement dans un rapport matière végétale / eau de 4 à 20% en poids/poids, plus préférentiellement dans un rapport de 5 à 15%, par exemple dans un rapport de 5, 10 ou 15% en poids/poids.

L'eau utilisée est une eau distillée, déminéralisée ou encore une eau riche en sels minéraux et/ou oligo-éléments, préférentiellement une eau distillée.

Préférentiellement, la matière végétale est broyée avant d'être mise en présence d'eau dans l'étape a). Le broyage est une action mécanique qui permet une meilleure extraction. Le broyage mécanique, suivi d'une lyse alcaline en présence d'EDTA favorise la complète destructuration de la membrane cellulaire et notamment de la membrane nucléaire.

On ajoute ensuite dans une étape b) de l'EDTA tétrasodique dans le mélange obtenu en a). Le pH à cette étape est basique et doit être ajusté, si nécessaire, à une valeur comprise entre 10,5 et 11 par l'ajout de soude (NaOH). Lors de l'étape b) il est essentiel de maintenir le pH basique entre 10,5 et 11. En effet, ce niveau de pH , associé à l'action de l'EDTA, provoque la destructuration de la membrane cellulaire, y compris la membrane nucléaire, la lyse des cellules et la dénaturation de l'ADN (les 2 brins de la double hélice sont séparés). Le contrôle du pH lors de l'étape b) montre que celui-ci reste basique et se stabilise entre 9 et 11.

La concentration en EDTA tétrasodique est préférentiellement comprise entre 2 et 15 mM, et plus préférentiellement de 10 mM.

Cette concentration est choisie pour optimiser le rendement d'extraction des ARN de petit poids moléculaire dans l'extrait final. L'EDTA tétrasodique va fragiliser, déstructurer les membranes pecto-cellulosiques des cellules végétales en séquestrant par complexation les ions divalents tels que les ions calcium qui forment des ponts ioniques entre les molécules de pectines entourant les microfibrilles de cellulose. Ceci a pour conséquence de favoriser la libération du contenu cellulaire au cours de l'extraction. L'étape de traitement par EDTA est essentielle pour enrichir l'extrait en ARN de petit poids moléculaire.

L'étape de traitement par l'EDTA dure préférentiellement au moins 1h, à une température comprise entre 20 et 80°C. Pendant cette étape, le mélange obtenu en a) est avantageusement mis sous agitation.

Dans une étape c), on ajuste ensuite le pH du mélange obtenu en b) à une valeur comprise entre 6 et 8.

Par exemple, on ajuste le pH par ajout d'une solution d'acide chlorhydrique (HCl) ou encore n'importe quel acide pouvant réguler le pH qui soit compatible avec une utilisation cosmétique tel que l'acide citrique ou lactique.

Cette étape d'acidification provoque la renaturation brutale de l'ADN (reappariement des brins du duplex). Néanmoins l'ADN chromosomique, très long, ne parvient pas à se réapparier complètement et forme des enchevêtrements insolubles. Au contraire, les petits ARN, beaucoup plus courts, restent en solution. L'ADN et les petits ARN sont ainsi séparés en deux phases distinctes ; une phase solide contenant entre autre l'ADN chromosomique, et une phase liquide contenant, entre autre, les petits ARN.

Dans une étape d) on purifie le mélange obtenu en c) de manière à éliminer la matière végétale et récupérer un extrait brut aqueux. Toute méthode connue par l'homme du métier pourra être utilisé. Préférentiellement le mélange obtenu en c) est centrifugé à faible vitesse, par exemple pendant au moins 10 min à 4000 g, de manière à sédimenter la matière végétale résiduelle dans le culot et récupérer un extrait brut aqueux dans le surnageant.

Avantageusement, de la terre de diatomée ou de la silice à une concentration de 10 à 20 g/kg sont ajoutées dans le mélange avant l'étape d) afin d'obtenir un culot bien compact lors de la centrifugation, ce qui permet d'obtenir un surnageant dépourvu de matières solides indésirables.

Dans une étape e) on contrôle le pH et on le réajuste à une valeur comprise entre 6 et 8. Préférentiellement le pH est réajusté à une valeur comprise entre 6 et 6,5, encore plus préférentiellement à 6,5. Le pH est réajusté par l'ajout d'une solution d'acide chlorhydrique (HCl) ou de soude (NaOH).

En effet, un pH inférieur à 6 peut entrainer la précipitation des acides nucléiques en général, donc celle des ARN de petit poids moléculaire d'une longueur d'au maximum 150 nucléotides.

L'étape d'ajustement du pH à l'étape e) du procédé selon l'invention est une étape indispensable à l'extraction optimale des ARN de petit poids moléculaire.

Avantageusement le réajustement du pH de l'étape e) est précédé par au moins une filtration de l'extrait brut aqueux obtenu en d). Préférentiellement des filtrations successives seront réalisées en abaissant le seuil de filtration de 20 à 50 µm puis de 0,1 à 0,3 µm.

Selon un mode de réalisation avantageux, le procédé selon l'invention comprend une étape additionnelle d'hydrolyse réalisée avant l'étape c), soit directement sur le mélange obtenu en b), soit sur le surnageant après centrifugation du mélange obtenu en b) et élimination de la matière végétale, par action d'au moins une enzyme choisie parmi une carbohydrase, une cellulase et/ou une protéase, préférentiellement une protéase, pendant au moins 1h, à une température comprise entre 45 et 65°C et à un pH ajusté en fonction de la ou des enzymes utilisées, généralement compris entre 6 et 8,5.

Lorsqu'une étape additionnelle d'hydrolyse est réalisée, le pH est ensuite réajusté si nécessaire entre 6 et 8 et préférentiellement entre 6 et 6,5 pour préserver les ARN de petit poids moléculaire extraits au cours des précédentes étapes et éviter qu'ils ne précipitent à cause d'un pH trop acide.

Dans ce mode de réalisation avantageux, le procédé comprend obligatoirement en outre une étape de désactivation de l'enzyme à une température comprise entre 65 et 80°C pendant au moins 1h, cette étape de désactivation étant réalisée directement après l'étape d'hydrolyse ou entre 2 des étapes de filtrations successives en e).

L'invention a pour deuxième objet un extrait aqueux de matière végétale, enrichi en petits ARN d'une longueur d'au maximum 150 nucléotides de l'invention est susceptible d'être obtenu par le procédé décrit ci-dessus.

Dans un mode de réalisation particulier, l'extrait aqueux enrichi en petits ARN d'une longueur d'au maximum 150 nucléotides de l'invention est obtenu par le procédé décrit ci-dessus.

Cet extrait ne contient pas d'ADN (acide désoxyribonucléique).

Un tel extrait aqueux enrichi en petits ARN d'une longueur d'au maximum 150 nucléotides comprend, avant dilution, en poids du poids total de l'extrait, de 5 à 60 g/kg d'extrait sec, et 10 à 1000 mg/kg de petits ARN d'une longueur d'au maximum 150 nucléotides. L'extrait susceptible d'être obtenu par le procédé selon l'invention comprend en outre de 0,5 à 30 g/kg de fragments protéiques et de 0,5 à 50 g/kg de sucres. Un tel extrait peut comprendre en outre 0,01 à 5 g/kg d'acides aminés et 0,01 à 4 g/kg de composés phénoliques.

L'extrait ainsi obtenu est considéré comme concentré. Il peut être ensuite dilué dans un solvant physiologiquement acceptable pour un usage cosmétique, de telle sorte que la concentration de l'extrait est alors ajustée à un poids en extrait sec particulier d'intérêt.

A titre d'exemples illustratifs et non limitatifs de solvants physiologiquement acceptables, on peut citer l'eau, le glycérol, l'éthanol, le propanediol ainsi que sa version naturelle appelée Zemea® issue du maïs, le butylène glycol, le dipropylène glycol, les diglycols éthoxylés ou propoxylés, les polyols cycliques ou tout mélange de ces solvants.

Préférentiellement, l'extrait susceptible d'être obtenu par le procédé selon l'invention est dilué dans un solvant tel que 30% de glycérol et de l'eau, et comprend en poids du poids total de l'extrait, de 5 à 35 g/kg d'extrait sec, et 10 à 500 mg/kg de petits ARN d'une longueur d'au maximum 150 nucléotides. Cet extrait dilué comprend en outre de 0,5 à 20 g/kg de fragments protéiques et 0,5 à 30 g/kg de sucres. Un tel extrait dilué peut comprendre en outre 0,01 à 3 g/kg d'acides aminés, 0,01 à 2 g/kg de composés phénoliques.

A titre illustratif, des exemples préférés de mode de réalisation du procédé selon l'invention sont décrits ci-dessous.

### Exemple 1 : Préparation d'un extrait de germes de riz (Oryza sativa) de la famille des Poaceae, enrichi en petits ARN

Un extrait aqueux enrichi en ARN de petit poids moléculaire (d'une longueur d'au maximum 150 nucléotides) est obtenu à partir de riz (*Oryza sativa*) de la famille des *Poaceae* (anciennement graminées).

Dans une première étape, 5% de germes de riz sous forme de poudre sont placés dans de l'eau distillée et 10 mM d'EDTA tétrasodique sont ajoutés, soit 50 g de poudre de germes de riz dans 1 kg d'eau distillée et 3,8 g d'EDTA tétrasodique. Le pH à cette étape doit être basique et compris entre 10,5 et 11 pour un enrichissement optimal de l'extrait en ARN de petit poids moléculaire.

Le mélange est mis sous agitation pendant 2h à température ambiante.

Une hydrolyse enzymatique est ensuite réalisée avec des protéases (d'alcalase® qui est une endopeptidase à serine et bromélaïne) ajoutées à 2% chacune par rapport à la matière végétale engagée, soit 1 g de chaque enzyme est ajouté dans le mélange. Le pH est ajusté entre 7,5 et 8.

Le mélange est ensuite chauffé 2h à 55°C, puis 2h à 80°C pour désactiver ces mêmes enzymes.

Le mélange est ensuite centrifugé avec des terres de diatomée (10 g pour 1 kg de mélange), 10 min à 4000 g, pour ôter la matière solide.

A l'issue de cette étape, le pH est contrôlé, avant dilution éventuelle, afin de le placer si nécessaire entre 6 et 6,5 et préserver les petits ARN de l'extrait.

Des filtrations séquentielles sur filtres de porosité décroissante sont alors réalisées afin de clarifier l'extrait végétal jusqu'à une filtration stérilisante à 0,2 µm.

D'une manière générale, on obtient un extrait aqueux de germe de riz de couleur jaune clair titrant de 20 à 50 g/kg d'extrait de poids sec, 3 à 15 g/kg de fragments protéiques, 5 à 30 g/kg de sucres, 1,5 à 3 g/kg d'acides aminés, 300 à 750 mg/kg de composés phénoliques et 50 à 400 mg/kg d'ARN de petit poids moléculaire d'une longueur d'au maximum 150 nucléotides.

Néanmoins, pour des germes de riz de l'espèce *Oryza sativa,* les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 23 g/kg d'extrait de poids sec. L'analyse physico-chimique montre que cet extrait présente une concentration de 4,8 g/kg de fragments protéiques, 8,2 g/kg de sucres, 2,3 g/kg d'acides aminés, 328 mg/kg de composés phénoliques et 132 mg/kg d'ARN de petit poids moléculaire d'une longueur d'au maximum 150 nucléotides. Cet extrait peut être par la suite dilué dans l'eau et conservé par l'ajout d'un solvant cosmétique tel que la glycerine et d'un conservateur tel que 1,5% de phenoxyethanol.

### Exemple 2 : Etude de l'influence du pH et de l'hydrolyse enzymatique dans la mise en œuvre du procédé

Afin d'étudier l'influence du pH dans la mise en œuvre du procédé selon l'invention, des essais d'extraction ont été effectués à différents pH et pour différents types de plantes.

Ces extractions ont notamment été réalisées sur le germe de riz (tel que décrit dans l'exemple 1). Des résultats comparables peuvent être obtenus de façon générale pour tous types de plantes considérés et plus particulièrement décrits selon l'invention.

5% de poudre de germes de riz sont placés dans de l'eau, soit 50 g de poudre de germes de riz dans 1 kg d'eau distillée, et 10 mM d'EDTA tétrasodique sont ajoutés (soit 3,8 g). Le pH a été ajusté soit à 7 soit à 11 en ajoutant entre 1 et 3 mL d'hydroxyde de sodium concentré, puis le mélange est mis sous agitation pendant 2h à température ambiante.

Des prélèvements sont alors effectués au cours du procédé d'extraction pour observer l'enrichissement en ARN de petit poids moléculaire pour chaque extrait après cette étape clé.

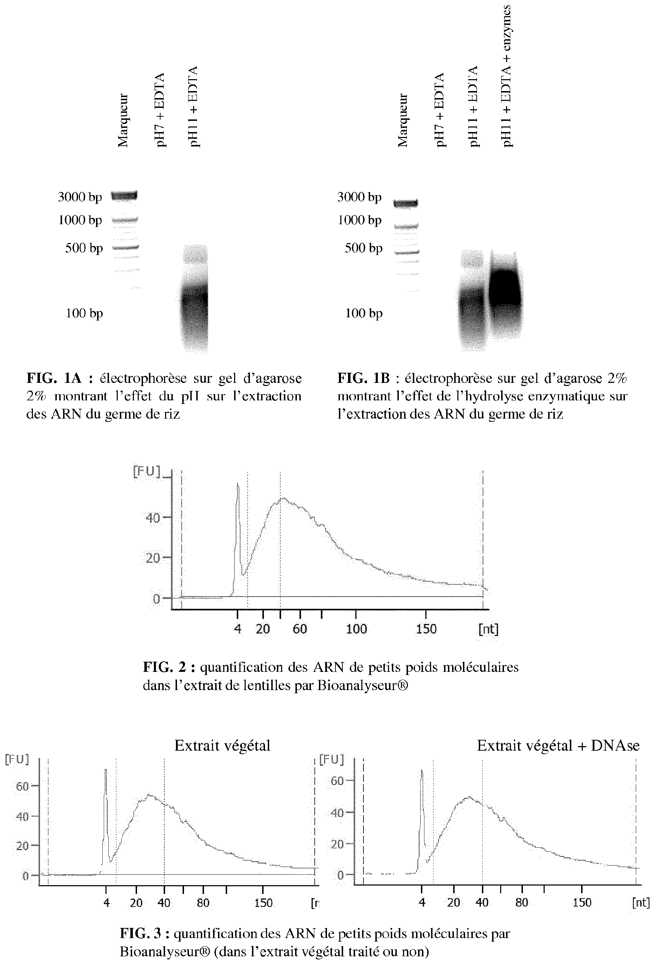

Une électrophorèse sur gel d'agarose à 2% est ainsi réalisée afin de visualiser la présence des ARN de petit poids moléculaire (Fig. 1A).

Tel qu'illustré par la Figure 1A, on peut observer que le pH optimal pour enrichir l'extrait en ARN de petit poids moléculaire est un pH basique de 11 ; en effet, à pH 7, l'extrait ne contient aucun ARN de petit poids moléculaire (absence de bande caractéristique).

L'utilisation d'un pH basique lors de l'étape de traitement par EDTA est donc une condition essentielle pour la mise en œuvre du procédé selon l'invention.

Dans un deuxième temps, une hydrolyse enzymatique est réalisée avec des protéases (d'alcalase® et bromélaïne) à 2% chacune par rapport à la matière végétale engagée, soit 1 g de chaque enzyme, dans le mélange. Le pH est ajusté entre 7,5 et 8, pH optimal d'activité des enzymes et optimal pour maintenir les ARN de petit poids moléculaire solubles dans le mélange, un pH inférieur à 6 les faisant précipiter.

Le mélange est ensuite chauffé 2h à 55°C, température optimale d'activité des enzymes, puis 2h à 80°C pour désactiver ces mêmes enzymes.

Le mélange est ensuite centrifugé avec des terres de diatomée (10 g pour 1 kg de mélange), 10 min à 4000 g, pour ôter la matière solide.

A l'issue de cette étape, le pH est contrôlé, avant dilution éventuelle, afin de le placer entre 6 et 6,5 et préserver les petits ARN de l'extrait.

Des filtrations séquentielles sur filtres de porosité décroissante sont alors réalisées afin de clarifier l'extrait végétal jusqu'à une filtration stérilisante de porosité 0,2 µm.

L'extrait final est visualisé par une électrophorèse sur gel d'agarose à 2% (Fig. 1B).

Tel qu'illustré par la Figure 1B, avantageusement, on constate que l'utilisation d'enzyme(s) améliore le rendement d'extraction des ARN de petit poids moléculaire dans l'extrait final.

Ce résultat a été observé sur différents types de végétaux extraits. Le fait que les enzymes potentialisent l'extraction d'ARN de petit poids moléculaire pourrait être dû au fait que des protéines sont souvent liées aux acides nucléiques ; la dégradation des protéines par des enzymes qui brisent les liaisons peptidiques des protéines créant ainsi des fragments protéiques de petites tailles (inférieurs à 10 kDa), permettrait de dissocier les ARN liés aux protéines et de les libérer augmentant ainsi leur rendement final dans l'extrait.

### Exemple 3 : Préparation d'un extrait de lentilles (Lens esculenta) de la famille des Fabaceae enrichi en petits ARN

Un extrait aqueux enrichi en ARN de petit poids moléculaire (d'une longueur d'au maximum 150 nucléotides) est obtenu à partir de lentilles (*Lens esculenta*).

Dans une première étape, les lentilles sont mises à germer la veille du procédé d'extraction, à savoir 40 g de lentilles recouvertes par de l'eau distillée.

Le lendemain, on rajoute de l'eau distillée afin d'obtenir l'équivalent de 4% de lentilles engagées dans le procédé d'extraction.

Les lentilles (40 g qsp 1 kg d'eau distillée) sont alors broyées et 10 mM d'EDTA tétrasodique (soit 3,8 g) est ajouté. Le pH est ajusté à 11 par ajout d'une solution de NaOH et le mélange est mis sous agitation pendant 2h à température ambiante.

Une filtration à large porosité est avantageusement réalisée pour ôter les débris solides.

Des protéases (2% de bromélaïne et 2% d'alcalase® par rapport à la matière végétale engagée) sont alors ajoutées dans le filtrat pour effectuer une hydrolyse enzymatique en prenant soin de laisser se solubiliser les enzymes avant d'ajuster le pH entre 7,5 et 8 par ajout d'une solution d'HCl.

Le filtrat est maintenu sous agitation 2h à 55°C, le temps que se déroule l'hydrolyse.

Des filtrations séquentielles sont alors réalisées sur des filtres de porosité décroissante de tailles comprise entre 20 et 50 µm puis 7 et 20 µm afin de clarifier l'extrait végétal brut aqueux.

L'extrait est ensuite mis à chauffer 1h à 80°C afin de désactiver les enzymes.

Les filtrations sont poursuivies jusqu'à une porosité de 0,3 à 0,4 µm.

A cette étape du procédé d'extraction, il est important de bien vérifier le pH, avant dilution éventuelle, afin de le placer avantageusement entre 6 et 6,5 et préserver les petits ARN de l'extrait.

D'une manière générale, on obtient un extrait aqueux de lentilles de couleur ambre-rouge titrant de 15 à 25 g/kg d'extrait de poids sec, 3 à 8 g/kg de fragments protéiques, 2 à 8 g/kg de sucres, 1 à 3 g/kg d'acides aminés, 300 à 750 mg/kg de composés phénoliques et 50 à 150 mg/kg d'ARN de petit poids moléculaire d'une longueur d'au maximum 150 nucléotides.

Néanmoins, pour des lentilles d'une même espèce (*Lens esculenta*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 15,4 g/kg d'extrait de poids sec, 6,9 g/kg de fragments protéiques, 2,1 g/kg de sucres, 1,4 g/kg d'acides aminés, 400 mg/kg de composés phénoliques et 96 mg/kg d'ARN de petit poids moléculaire d'une longueur d'au maximum 150 nucléotides.

L'extrait est alors dilué dans de l'eau ou dans un solvant physiologiquement acceptable comprenant par exemple de l'eau et 30% de glycérol, de telle sorte que l'extrait final soit ajusté à 10 g/kg d'extrait de poids sec.

L'analyse physico-chimique montre qu'après dilution cet extrait présente une concentration en fragments protéiques de 5,1 g/kg, en sucres de 1,5 g/kg, en acides aminés de 0,8 g/kg, en composés phénoliques de 280 mg/kg et en ARN de petit poids moléculaire (d'une longueur d'au maximum 150 nucléotides) de 70 mg/kg tel qu'illustré plus particulièrement dans la Figure 2 par la quantification des ARN de bas poids moléculaires au Bioanalyseur® (Agilent).

Le Bioanalyseur® est un appareil qui permet de réaliser des électrophorèses miniaturisées grâce à des puces électroniques spécifiques de l'analyse des acides nucléiques telle que celle des ARN de petit poids moléculaire. Il permet de déterminer la taille et la concentration contenues dans un extrait à partir de quelques microlitres. Le résultat se présente sous forme d'un graphique avec une unité arbitraire de fluorescence en ordonnée (FU) et en abscisse le nombre de nucléotides (nt). Un marqueur interne est ajouté à chaque analyse (pic à 4 nt sur la figure 2), et sert de contrôle interne pour valider le bon déroulement de l'analyse.

### Exemple 4 : Test relatif à l'absence d'ADN dans l'extrait selon l'exemple 3 (lentilles)

Afin de vérifier que l'acide nucléique obtenu dans les extraits selon l'invention est bien de l'ARN, et plus particulièrement de l'ARN de petit poids moléculaire (d'une longueur d'au maximum 150 nucléotides), et non de l'ADN, un test utilisant une DNAse (OPTIZYME™ Fisher Bioreagent) qui dégrade spécifiquement l'ADN a été réalisé en suivant le protocole recommandé par le fournisseur.

Une solution contrôle contenant 500 µg/mL d'ADN de saumon (Sigma, 31149-10g-F) et une autre solution contrôle contenant 500 µg/mL d'ARN de petit poids moléculaire issus de la levure Torula (Sigma R6625-25G) ont été réalisées. Le volume réactionnel contient pour 1 µg d'ADN ou d'ARN, 1 µL de RNAse, 1 µL de tampon 10X et complété jusqu'à 10 µL avec de l'eau DEPC (diéthylpyrocarbonate) 0,1% v/v. Le mélange réactionnel est ensuite mis à incuber 30 min à 37°C, condition optimale de réaction de la DNAse. L'enzyme est ensuite désactivée en rajoutant 50 mM d'EDTA tétrasodique et en chauffant 10 min à 65°C.

Pour visualiser le profil des solutions contrôle et de l'extrait selon l'exemple 3 après action de la DNAse, les petits ARN ont été quantifiés au Bioanalyseur®, avant et après traitement par la DNAse. Les profils obtenus avec ou sans traitement à la DNAse de l'extrait végétal sont identiques, la quantité restant la même soit environ 30 mg/kg, tel qu'illustré à la Figure 3. Un marqueur interne est ajouté à chaque analyse (pic à 4 nt sur la figure 3), et sert de contrôle interne pour valider le bon déroulement de l'analyse.

Le test à la DNAse, enzyme qui dégrade spécifiquement l'ADN et non l'ARN, démontre que l'acide nucléique de l'extrait selon l'exemple 3 est toujours présent après le traitement à la DNAse. Il s'agit donc bien d'ARN, en particulier d'ARN de petit poids moléculaire, d'une longueur d'au maximum 150 nucléotides, et non d'ADN.

### Exemple 5 : Préparation d'un extrait de gombo (Hibiscus esculentus) de la famille des Malvaceae, enrichi en petits ARN

Un extrait aqueux enrichi en ARN de petit poids moléculaire (d'une longueur d'au maximum 150 nucléotides) est obtenu à partir de fruits de gombo de l'espèce *Hibiscus esculentus.*

Dans une première étape, après décongélation, 10% de fruits *d'Hibiscus esculentus* sont mélangés dans de l'eau distillée soit par exemple 100 g de fruits dans 1 kg d'eau distillée, puis sont broyés pendant 10 minutes avec ajout d'EDTA tétrasodique à une concentration finale de 10 mM soit pour 1kg, 3,8 g. Le pH à cette étape est compris entre 10,5 et 11, pH optimal pour enrichir l'extrait en petits ARN.

Ce mélange est ensuite mis sous agitation pendant 2h à 45°C. Même si la température à ce stade peut varier de 20°C à 80°C, pour cette espèce une température de 45°C s'avère être la température qui permet d'obtenir les meilleurs résultats en terme d'enrichissement de l'extrait aqueux final en ARN de petit poids moléculaire.

A l'issue des 2h, de la terre de diatomée (ou de la silice) est ajoutée à une concentration de 10 g/kg et le mélange est agité encore 10 min, suivi d'une centrifugation à 4000 g pendant 10 min.

Le surnageant est alors récolté. Cet extrait brut contient notamment des fragments protéiques, des sucres, et des ARN de petit poids moléculaire.

Des protéases (2% de bromélaïne et 2% d'alcalase® par rapport à la quantité de matière végétale engagée) sont ajoutées pour effectuer une hydrolyse enzymatique, en prenant soin de laisser se solubiliser les enzymes avant d'ajuster le pH entre 7,5 et 8.

La solution brute est maintenue sous agitation 2h à 55°C, le temps que se déroule l'hydrolyse. L'hydrolyse enzymatique va permettre d'obtenir des fragments protéiques de petit poids moléculaire (inférieurs à 10 kDa, les protéines de hauts poids moléculaires pouvant être allergènes). De tels fragments protéiques ainsi obtenus peuvent en outre présenter une activité biologique intéressante au niveau de la peau.

Afin de commencer la clarification de l'extrait brut, des filtrations séquentielles sont alors réalisées sur des filtres de porosité décroissante de tailles comprise entre 20 et 50 µm puis 7 et 20 µm, suivies d'une étape de chauffage à haute température de l'extrait à 80°C sur la nuit. Cette étape permet de désactiver les protéases qui sous l'action d'une forte chaleur se dénaturent et deviennent alors inactives.

Les filtrations sont ensuite poursuivies jusqu'à une filtration stérilisante de 0,1 à 0,3 µm.

L'extrait végétal doit avoir un pH final compris entre 6 et 8 pour éviter que les ARN de petit poids moléculaire ne précipitent. A cette étape du procédé d'extraction, il faut bien vérifier le pH, avant dilution éventuelle, afin de le placer encore plus préférentiellement entre 6 et 6,5.

De manière générale, on obtient un extrait aqueux de couleur jaune pâle titrant de 10 à 20 g/kg d'extrait de poids sec, 2 à 5 g/kg de fragments protéiques, 2,5 à 5 g/kg de sucres, 0,1 à 2 g/kg d'acides aminés, 0,2 à 3 g/kg de composés phénoliques, et de 10 à 100 mg/kg d'ARN de petit poids moléculaire d'une longueur d'au maximum 150 nucléotides.

Néanmoins, pour des fruits de gombo d'une même espèce *(Hibiscus esculentus),* les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 13,3 g/kg d'extrait de poids sec, 3,2 g/kg de fragments protéiques, 3,9 g/kg de sucres, 790 mg/kg d'acides aminés, 490 mg/kg de composés phénoliques, et 60 mg/kg d'ARN de petit poids moléculaire d'une longueur d'au maximum 150 nucléotides.

L'extrait est alors dilué dans un solvant physiologiquement acceptable comprenant par exemple de l'eau et 30% de glycérol, de telle sorte que l'extrait final soit ajusté à 10 g/kg d'extrait de poids sec.

L'analyse physico-chimique montre qu'après dilution l'extrait présente une concentration en fragments protéiques de 2,5 g/kg, en sucres de 2,7 g/kg, en acides aminés de 520 mg/kg, en composés phénoliques de 320 mg/kg et de 35 mg/kg d'ARN de petit poids moléculaire d'une longueur d'au maximum 150 nucléotides.

### Exemple 6 : Mise en évidence du rôle d'une étape de traitement par EDTA dans la mise en œuvre d'un procédé d'extraction de petits ARN à partir de gombo (Hibiscus esculentus)

Dans le but de mettre en évidence le rôle d'une étape de traitement par EDTA lors de l'extraction des ARN de petit poids moléculaire, un extrait de fruits d'*Hibiscus esculentus* a été obtenu, en modifiant certaines étapes essentielles du procédé selon l'invention, ne permettant pas d'enrichir l'extrait en ARN de petit poids moléculaire.

15% de fruits décongelés d'*Hibiscus esculentus* sont mélangés avec de l'eau distillée puis sont broyés, soit 150 g de fruits dans 1 kg d'eau distillée.

Des protéases sont ensuite ajoutées de façon séquentielle : 2% d'alcalase® par rapport à la matière végétale engagée (soit 3 g) à pH 8 pendant 2h à 55°C (condition optimale pour cette enzyme), puis 2% de bromélaïne (soit 3 g) à pH ajusté à une valeur comprise entre 4 et 4,5 pendant 2h à 55°C.

Ce mélange est alors centrifugé pour ôter les débris solides.

Afin de commencer la clarification de l'extrait brut, des filtrations séquentielles sont alors réalisées sur des filtres de porosité décroissante de tailles comprise entre 20 et 50 µm puis 7 et 20 µm, suivies d'une étape de chauffage à haute température de l'extrait à 80°C sur la nuit.

Les filtrations sont poursuivies jusqu'à une filtration stérilisante de 0,1 à 0,3 µm.

L'extrait aqueux obtenu est ensuite dilué dans un solvant physiologiquement acceptable comprenant par exemple de l'eau et 30% de glycérol, pour atteindre 9,6 g/kg d'extrait de poids sec. Le pH de l'extrait final est compris entre 4 et 4,5.

L'analyse physico-chimique montre que l'extrait végétal final, après dilution, présente une concentration en fragments protéiques de 2,2 g/kg, en sucres de 3,8 g/kg, 550 mg/kg d'acides aminés et 243 mg/kg de composés phénoliques.

L'analyse au Bioanalyseur® indique que la concentration en ARN de petit poids moléculaire est nulle pour cet extrait. Ce résultat démontre qu'un extrait obtenu à partir d'un procédé d'extraction ne présentant pas d'étape de traitement par EDTA ne contient pas d'ARN de petit poids moléculaire. L'étape de traitement par EDTA est par conséquent essentielle pour obtenir un extrait riche en ARN de petit poids moléculaire selon l'invention.

### Exemple 7 : Préparation d'un extrait de baobab (Adansonia digitata) de la famille des Bombacaceae enrichi en petits ARN

Un extrait aqueux enrichi en ARN de petit poids moléculaire (d'une longueur d'au maximum 150 nucléotides) est obtenu à partir de baobab de l'espèce *Adansonia digitata.*

Dans une première étape, 5% de tourteau de graine de baobab (*Adansonia digitata*)) sont broyés à sec ou directement dans de l'eau contenant de l'EDTA tétrasodique à une concentration finale de 10 mM, soit 50 g de tourteau de baobab dans 1 kg d'eau distillée auquel on ajoute 3,8 g d'EDTA tétrasodique. Le pH à cette étape est basique et plus particulièrement compris entre 10,5 et 11, pH optimal pour enrichir l'extrait en ARN de petit poids moléculaire.

Le mélange est mis sous agitation pendant 2h à 58°C.

Pour cette espèce, avantageusement une étape d'hydrolyse avec une enzyme protéolytique est réalisée : 2% de papaïne sont ajoutés par rapport à la quantité de matière végétale engagée (soit 1 g). Le pH du mélange est ajusté entre 7 et 8 si besoin et mis sous agitation pendant 2h à 58°C, conditions optimales pour cette enzyme.

Ensuite, le pH est ajusté à 8 puis l'extrait est centrifugé 10 min à 4000 g pour ôter la matière solide.

Des filtrations séquentielles sont alors réalisées sur des filtres de porosité décroissante de taille comprise entre 20 et 50 µm puis 7 et 20 µm afin de clarifier l'extrait végétal.

L'extrait est ensuite mis à chauffer à 80°C entre 8 et 12 heures afin de désactiver thermiquement l'enzyme.

Les filtrations sont poursuivies jusqu'à une porosité de 0,3 à 0,4 µm. A cette étape du procédé d'extraction, il faut bien vérifier le pH, avant dilution éventuelle, afin de le placer entre 6 et 6,5 et préserver les petits ARN de l'extrait. Un pH acide peut entrainer la précipitation des acides nucléiques en général donc également celle des ARN de petit poids moléculaire.

De manière générale, on obtient un extrait aqueux de baobab de couleur ambre-rouge titrant de 15 à 25 g/kg d'extrait de poids sec, 3 à 8 g/kg de fragments protéiques, 2 à 8 g/kg de sucres, 0,05 à 1 g/kg d'acides aminés, 0,05 à 1 g/kg de composés phénoliques et 10 à 80 mg/kg d'ARN de petit poids moléculaire d'une longueur d'au maximum 150 nucléotides.

Néanmoins, des extraits obtenus à partir de l'espèce *Adansonia digitata* peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 17 g/kg d'extrait de poids sec, 5,3 g/kg de fragments protéiques, 5,4 g/kg de sucres, 650 mg/kg d'acides aminés, 441 mg/kg de composés phénoliques, et 57 mg/kg d'ARN de petit poids moléculaire d'une longueur d'au maximum 150 nucléotides.

L'extrait est alors dilué dans un mélange d'eau et 30% de glycérol et 1,5% de phénoxyethanol sont ajoutés, ce qui permet d'obtenir un extrait final à 12 g/kg d'extrait de poids sec.

L'analyse physico-chimique montre qu'après dilution cet extrait présente une concentration en fragments protéiques de 2,9 g/kg, en sucres de 4 g/kg, en acides aminés de 400 mg/kg, en composés phénoliques de 310 mg/kg, et de 41 mg/kg d'ARN de petit poids moléculaire d'une longueur d'au maximum 150 nucléotides.

### Exemple 8 : Mise en évidence du rôle d'une étape de traitement par EDTA pour la mise en œuvre d'un procédé d'extraction de petits ARN à partir de baobab (Adansonia digitata)

Dans le but de mettre en évidence le rôle d'une étape de traitement par EDTA lors de l'extraction des ARN de petit poids moléculaire, un extrait de baobab (*Adansonia digitata*) a également été obtenu, en modifiant certaines étapes essentielles du procédé selon l'invention, ne permettant pas d'enrichir l'extrait en ARN de petit poids moléculaire.

Dans une première étape, 10% de tourteau de graine de baobab (*Adansonia digitata*) sont broyés puis de l'eau est ajoutée, soit 100 g de tourteau de baobab dans 1 kg d'eau distillée.

Pour cette espèce, une hydrolyse avec une enzyme protéolytique est réalisée : 2% de papaïne sont ajoutés par rapport à la quantité de matière végétale engagée soit 2 g. Le pH est ajusté entre 7 et 8, et le mélange est mis sous agitation pendant 2h à 58°C, conditions optimales pour l'activité de l'enzyme. Après cette période, le pH est descendu à 4,5, pH couramment utilisé pour les ingrédients cosmétiques.

Ensuite, l'extrait est centrifugé 10 min à 4000 g pour ôter la matière solide. L'extrait est ensuite mis à chauffer à 80°C entre 8 et 12h afin de désactiver l'enzyme par haute température.

Des filtrations séquentielles sont alors réalisées sur des filtres de porosité décroissante de taille comprise entre 20 et 50 µm puis jusqu'à une porosité de 0,3 à 0,4 µm.

On obtient alors un extrait aqueux de couleur jaune clair titrant 12,5 g/kg d'extrait de poids sec, 5,8 g/kg de fragments protéiques, 7,6 g/kg de sucres, 540 mg/kg d'acides aminés, et 440 mg/kg de composés phénoliques.

L'extrait est alors dilué dans de l'eau et du glycérol de façon à obtenir un extrait final à 30% de glycérol et ajusté à 10 g/kg d'extrait de poids sec.

L'analyse physico-chimique montre qu'après dilution l'extrait végétal présente une concentration en fragments protéiques de 3,25 g/kg, en sucres de 5,1 g/kg, en acides aminés de 310 mg/kg, et en composés phénoliques de 250 mg/kg. Dans ces conditions d'extraction (absence de traitement par EDTA), l'analyse au Bioanalyseur® indique que la concentration en ARN de petit poids moléculaire est nulle pour cet extrait. Ce résultat confirme qu'un extrait obtenu à partir d'un procédé d'extraction en l'absence de traitement par EDTA (à pH basique) ne contient pas d'ARN de petit poids moléculaire. L'étape de traitement par EDTA est essentielle pour obtenir un extrait riche en ARN de petit poids moléculaire selon l'invention.

### Exemple 9 : Etude de l'influence du pH final dans la préparation d'extraits de gombo (Hibiscus esculentus) et de baobab (Adansonia digitata)

Le procédé d'extraction est réalisé dans les mêmes conditions opératoires que les exemples 5 et 7, pour enrichir un extrait en ARN de petit poids moléculaire, hormis l'étape finale d'ajustement du pH.

Ce procédé d'extraction est réalisé avec étape de traitement par EDTA tétrasodique, suivi de l'étape d'hydrolyse enzymatique, mais avec un ajustement final de l'extrait à un pH acide compris entre 4 et 4,5 au lieu d'un pH compris entre 6 et 8.

Ceci entraine la précipitation des ARN de petit poids moléculaire, résultats confirmés par l'analyse au Bioanalyseur® qui donne une concentration nulle en ARN de petit poids moléculaire pour chacun des extraits de gombo (*Hibiscus esculentus*) et de baobab (*Adansonia digitata*) ainsi obtenus.

### Exemple 10 : Préparation d'un extrait de courge (Cucurbita pepo) de la famille des Cucurbitaceae enrichi en petits ARN

Un extrait aqueux enrichi en ARN de petit poids moléculaire (d'une longueur d'au maximum 150 nucléotides) est obtenu à partir de courge (*Cucurbita pepo*) de la famille des cucurbitacées.

Dans une première étape, 10% de tourteau de graine de courge sont mélangés avec de l'eau et de l'EDTA tétrasodique est ajouté pour obtenir une concentration finale de 10 mM, soit 100 g de tourteau de courge dans 1 kg d'eau distillée et 3,8 g d'EDTA tétrasodique.

Le pH à cette étape doit être basique et plus particulièrement compris entre 10,5 et 11 pour un enrichissement optimal de l'extrait en ARN de petit poids moléculaire.

Le mélange est mis sous agitation pendant 2h à 45°C.

Une hydrolyse enzymatique est ensuite réalisée avec des protéases : 2% d'alcalase® et 4% de papaïne par rapport à la quantité de matière végétale engagée, soit 2 g d'alcalase® et 4 g de papaïne, sont ainsi ajoutés dans le mélange. Le pH est ajusté entre 7,5 et 8, pH optimal d'activité pour ces deux enzymes, ce pH restant optimal pour maintenir les ARN de petit poids moléculaire solubles dans le mélange, un pH inférieur à 6 pouvant les faire précipiter.

Le mélange est ensuite chauffé 2h à 50°C, température optimale d'activité des enzymes puis 2h à 80°C pour désactiver ces mêmes enzymes.

Le mélange est ensuite centrifugé avec des terres de diatomée (10 g pour 1 kg de mélange), 10 min à 4000 g, pour ôter la matière solide.

A l'issue de cette étape, le pH est contrôlé, avant dilution éventuelle, afin de le placer si besoin entre 6 et 6,5 et préserver les petits ARN de l'extrait.

Des filtrations séquentielles sont alors réalisées sur des filtres de porosité décroissante de taille comprise entre 20 et 50 µm puis 7 et 20 µm afin de clarifier l'extrait végétal. Puis, les filtrations se poursuivent jusqu'à la filtration stérilisante à 0,2 µm.

De manière générale, on obtient un extrait aqueux de couleur jaune clair titrant de 20 à 50 g/kg d'extrait de poids sec, 3 à 25 g/kg de fragments protéiques, 1 à 10 g/kg de sucres, et 50 à 250 mg/kg d'ARN de petit poids moléculaire d'une longueur d'au maximum 150 nucléotides.

Néanmoins, pour des courges d'une même espèce (*Cucurbita pepo*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 37,3 g/kg d'extrait de poids sec, 18,4 g/kg de fragments protéiques, 3,8 g/kg de sucres et 168 mg/kg d'ARN de petit poids moléculaire d'une longueur d'au maximum 150 nucléotides.

L'extrait peut être alors dilué dans un solvant physiologiquement acceptable comprenant par exemple de l'eau et 30% de glycérol.

### Exemple 11 : Préparation d'un extrait de quinoa (Chenopodium quinoa), de la famille des chénopodiacées ou pseudo céréale, enrichi en petits ARN

Un extrait aqueux enrichi en ARN de petit poids moléculaire (d'une longueur d'au maximum 150 nucléotides) est obtenu à partir de quinoa *Chenopodium quinoa,* de la famille des chénopodiacées ou pseudo céréale.

Dans une première étape, les graines de quinoa germées (10% de poids final, soit 100 g) sont engagés dans le procédé d'extraction et mélangés dans 1 kg d'eau distillée puis sont broyées, et de l'EDTA tétrasodique est ajouté pour obtenir une concentration finale de 10 mM soit 3,8 g. Le pH à cette étape doit être basique et compris entre 10,5 et 11, le pH étant ainsi ajusté avec du NaOH, pour un enrichissement optimal de l'extrait selon l'invention en ARN de petit poids moléculaire d'une longueur d'au maximum 150 nucléotides. Le mélange est mis sous agitation pendant 2h à 55°C.

Une hydrolyse enzymatique est ensuite réalisée avec des protéases : 2% d'alcalase® et 2% de bromélaïne sont ajoutés dans le mélange (par rapport à la matière engagée). Le pH est ajusté entre 7,5 et 8, pH optimal d'activité pour ces deux enzymes, ce pH restant optimal pour maintenir les ARN de petit poids moléculaire solubles dans le mélange, un pH inférieur à 6 les faisant précipiter.

Le mélange est chauffé 2h à 45°C, température optimale d'activité des enzymes.

Le mélange est ensuite centrifugé avec des terres de diatomée (10 g pour 1 kg de mélange), 10 min à 4000 g, pour ôter la matière solide.

Puis, le filtrat est chauffé 2h à 80°C pour désactiver ces enzymes.

Des filtrations séquentielles sont alors réalisées sur des filtres de porosité décroissante de taille comprise entre 20 et 50 µm puis 7 et 20 µm afin de clarifier l'extrait végétal.

Après cette étape de filtration, on peut effectuer une dilution de l'extrait dans préférentiellement de l'eau et du glycérol de façon à obtenir un extrait final contenant 30% de glycérol.

Puis, les filtrations se poursuivent jusqu'à la filtration stérilisante à 0,2 µm.

De manière générale, on obtient un extrait de couleur jaune clair titrant de 20 à 50 g/kg d'extrait de poids sec, 3 à 15 g/kg de fragments protéiques, 10 à 30 g/kg de sucres, 0,5 à 5 g/kg d'acides aminés, 100 à 700 mg/kg de composés phénoliques et de 50 à 250 mg/kg d'ARN de petit poids moléculaire d'une longueur d'au maximum 150 nucléotides.

Des résultats similaires peuvent être obtenus à partir de graines de quinoa non germées ou encore de farine de quinoa.

Néanmoins, pour du quinoa d'une même espèce (*Chenopodium quinoa*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 33 g/kg d'extrait de poids sec, 9,5 g/kg de fragments protéiques, 21,6 g/kg de sucres, 1,8 g/kg d'acides aminés, 364 mg/kg de composés phénoliques, et 173 mg/kg d'ARN de petit poids moléculaire d'une longueur d'au maximum 150 nucléotides.

L'extrait peut alors être dilué dans un solvant physiologiquement acceptable comprenant par exemple de l'eau et 30% de glycérol.

### Exemple 12 : Préparation d'un extrait de rose (Rosa centifolia) de la famille des Rosaceae enrichi en petits ARN

Un extrait aqueux enrichi en ARN de petit poids moléculaire (d'une longueur d'au maximum 150 nucléotides) est obtenu à partir de rose (*Rosa centifolia*) de la famille des Rosacées. Dans cet exemple, la fleur fraîche entière est utilisée.

Dans une première étape, 10% de roses sont mélangés dans de l'eau distillée, soit par exemple 100 g de fleurs complété à 1 kg d'eau distillée, puis sont broyés pendant 10 minutes avec ajout d'EDTA tétrasodique à une concentration finale de 10 mM soit pour 1kg, 3,8 g. Le pH à cette étape est compris entre 10,5 et 11, pH optimal pour enrichir l'extrait en petits ARN.

Ce mélange est ensuite mis sous agitation pendant 1h à 80°C. Même si la température à ce stade peut varier de 50°C à 80°C, pour cette espèce une température de 80°C s'avère être la température qui permet d'obtenir les meilleurs résultats en terme d'enrichissement de l'extrait aqueux final en ARN de petit poids moléculaire.

A l'issue de cette étape, des filtrations séquentielles sont alors réalisées sur des filtres de porosité décroissante de taille comprise entre 20 et 50 µm puis 7 et 20 µm afin d'ôter la matière solide puis de clarifier l'extrait végétal.

A cette étape, le pH est contrôlé, afin de le placer si besoin entre 6 et 6,5 et préserver les petits ARN de l'extrait.

Puis, les filtrations se poursuivent jusqu'à la filtration stérilisante à 0,2 µm.

De manière générale, on obtient un extrait aqueux de couleur ambré titrant de 5 à 20 g/kg d'extrait de poids sec, 1 à 10 g/kg de fragments protéiques, 1 à 10 g/kg de sucres, 0,5 à 2 g/kg de composés phénoliques et 20 à 200 mg/kg d'ARN de petit poids moléculaire d'une longueur d'au maximum 150 nucléotides.

Néanmoins, pour des roses d'une même espèce (*Rosa centifolia*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 11.3 g/kg d'extrait de poids sec, 5.1 g/kg de fragments protéiques, 2.6 g/kg de sucres, 1 g/kg de composés phénoliques et 96 mg/kg d'ARN de petit poids moléculaire d'une longueur d'au maximum 150 nucléotides.

L'extrait peut être alors dilué avec par exemple 30% de glycérol ce qui permet d'obtenir un extrait final à 8 g/kg d'extrait de poids sec.

L'analyse physico-chimique montre qu'après dilution cet extrait présente une concentration en fragments protéiques de 4.6 g/kg, en sucres de 2 g/kg, en acides aminés de 280 mg/kg, en composés phénoliques de 800 mg/kg, et de 83 mg/kg d'ARN de petit poids moléculaire d'une longueur d'au maximum 150 nucléotides.

### Exemple 13 : Préparation d'un extrait de fleurs de bigaradier (Citrus aurantium) de la famille des Rutaceae enrichi en petits ARN

Un extrait aqueux enrichi en ARN de petit poids moléculaire (d'une longueur d'au maximum 150 nucléotides) est obtenu à partir de fleurs de bigaradier (*Citrus aurantium*) de la famille des Rutacées. Dans cet exemple, la fleur fraiche et entière est utilisée.

Dans une première étape, 5% de fleurs de bigaradier sont mélangées dans de l'eau distillée, soit par exemple 50 g de fleurs complété à 1 kg d'eau distillée, puis sont broyées pendant 5 minutes avec ajout d'EDTA tétrasodique à une concentration finale de 10 mM soit pour 1kg, 3,8 g. Le pH à cette étape est compris entre 10,5 et 11, pH optimal pour enrichir l'extrait en petits ARN.

Ce mélange est ensuite mis sous agitation pendant 1h à 45°C. Même si la température à ce stade peut varier de 25°C à 50°C, pour cette espèce une température de 45°C s'avère être la température qui permet d'obtenir les meilleurs résultats en terme d'enrichissement de l'extrait aqueux final en ARN de petit poids moléculaire.

A l'issue de cette étape, des filtrations séquentielles sont alors réalisées sur des filtres de porosité décroissante de taille comprise entre 20 et 50 µm puis 7 et 20 µm, afin d'ôter la matière solide puis de clarifier l'extrait végétal.

A cette étape, le pH est contrôlé, afin de le placer si besoin entre 6 et 6,5 et préserver les petits ARN de l'extrait.

Les filtrations sont ensuite poursuivies jusqu'à une porosité de 0,3 à 0,5 µm.

De manière générale, on obtient un extrait aqueux de couleur ambré titrant de 5 à 20 g/kg d'extrait de poids sec, 1 à 10 g/kg de fragments protéiques, 1 à 10 g/kg de sucres, 200 à 1000 mg/kg de composés phénoliques et 10 à 100 mg/kg d'ARN de petit poids moléculaire d'une longueur d'au maximum 150 nucléotides.

Néanmoins, pour des fleurs d'une même espèce (*Citrus aurantium*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 11.8 g/kg d'extrait de poids sec, 4.5 g/kg de fragments protéiques, 3.8 g/kg de sucres, 560 mg/kg de composés phénoliques et 20 mg/kg d'ARN de petit poids moléculaire d'une longueur d'au maximum 150 nucléotides.

L'extrait peut alors être dilué dans un solvant physiologiquement acceptable tel que de l'eau ou du glycérol.

### Exemple 14 : Préparation d'un extrait de lis (Lilium candidum) enrichi en petits ARN

Un extrait aqueux enrichi en ARN de petit poids moléculaire (d'une longueur d'au maximum 150 nucléotides) est obtenu à partir de bulbe de lis blanc (*Lilium candidum*) de la famille des Liliacées.

Dans une première étape, 15% de bulbes de lis sont placés dans de l'eau distillée, soit par exemple 150 g de bulbes dans 1 kg d'eau distillée contenant de l'EDTA tétrasodique à une concentration finale de 10 mM soit pour 1kg, 3,8 g, puis on effectue le broyage de la soltuion pendant 5 minutes. Le pH à cette étape est compris entre 10,5 et 11, pH optimal pour enrichir l'extrait en petits ARN.

Ce mélange est ensuite mis sous agitation pendant 30 minutes à 65°C. Même si la température à ce stade peut varier de 50°C à 80°C et le temps d'agitation de 30 minutes à 1h, pour cette espèce une température de 65°C pendant 30 minutes s'avère être les conditions qui permettent d'obtenir les meilleurs résultats en terme d'enrichissement de l'extrait aqueux final en ARN de petit poids moléculaire.

A l'issue de cette étape, des filtrations séquentielles sont alors réalisées sur des filtres de porosité décroissante de taille comprise entre 20 et 50 µm puis 7 et 20 µm, afin d'ôter la matière solide puis de clarifier l'extrait végétal.

A cette étape, le pH est contrôlé, afin de le placer si besoin entre 6 et 6,5 et préserver les petits ARN de l'extrait.

Les filtrations sont poursuivies jusqu'à une porosité de 2 à 4 µm. L'extrait peut ensuite être conservé grâce à l'ajout de 30 % de glycérol et 1,5 % de phénoxyéthanol. Les filtrations sont poursuivies jusqu'à une porosité de 0,2 à 0,3 µm.

De manière générale, on obtient un extrait aqueux de couleur jaune titrant de 10 à 25 g/kg d'extrait de poids sec, 0,5 à 5 g/kg de fragments protéiques, 2 à 15 g/kg de sucres, 100 à 500 mg/kg de composés phénoliques et 10 à 100 mg/kg d'ARN de petit poids moléculaire d'une longueur d'au maximum 150 nucléotides.

Néanmoins, pour des lis d'une même espèce (*Lilium candidum*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 16.3 g/kg d'extrait de poids sec, 1,5 g/kg de fragments protéiques, 5.3 g/kg de sucres, 200 mg/kg de composés phénoliques et 20 mg/kg d'ARN de petit poids moléculaire d'une longueur d'au maximum 150 nucléotides.

L'extrait peut alors être dilué dans un solvant physiologiquement acceptable tel que de l'eau ou du glycérol.

### Exemple 15 : Préparation d'un extrait de lis (Lilium tigrinum) de la famille des Liliaceae enrichi en petits ARN

Un extrait aqueux enrichi en ARN de petit poids moléculaire (d'une longueur d'au maximum 150 nucléotides) est obtenu à partir de bulbe de lis tigré (*Lilium tigrinum*) de la famille des Liliacées.

Dans une première étape, après lavage et décongélation, 10% de bulbes de lis sont mélangés dans de l'eau distillée, soit par exemple 100 g de bulbes dans 1 kg d'eau distillée contenant de l'EDTA tétrasodique à une concentration finale de 10 mM soit pour 1kg, 3,8 g puis le mélange est broyé pendant 5 minutes. Le pH à cette étape est compris entre 10,5 et 11, pH optimal pour enrichir l'extrait en petits ARN.

Ce mélange est ensuite mis sous agitation pendant 1 heure à 80°C. Même si la température à ce stade peut varier de 50°C à 80°C et le temps d'agitation de 30 minutes à 1h, pour cette espèce une température de 80°C pendant 1 heure s'avère être les conditions qui permettent d'obtenir les meilleurs résultats en terme d'enrichissement de l'extrait aqueux final en ARN de petit poids moléculaire.

A l'issue de cette étape, des filtrations séquentielles sont alors réalisées sur des filtres de porosité décroissante de taille comprise entre 20 et 50 µm puis 7 et 20 µm, afin d'ôter la matière solide puis de clarifier l'extrait végétal.

A cette étape, le pH est contrôlé, afin de le placer si besoin entre 6 et 6,5 et préserver les petits ARN de l'extrait. Les filtrations sont poursuivies jusqu'à une porosité de 0,2 à 0,3 µm.

De manière générale, on obtient un extrait aqueux de couleur jaune titrant de 10 à 25 g/kg d'extrait de poids sec, 0,5 à 5 g/kg de fragments protéiques, 5 à 20 g/kg de sucres, 100 à 500 mg/kg de composés phénoliques et 10 à 100 mg/kg d'ARN de petit poids moléculaire d'une longueur d'au maximum 150 nucléotides.

Néanmoins, pour des lis d'une même espèce (*Lilium tigrinum*), les extraits obtenus peuvent présenter une variabilité importante en fonction de facteurs tels que le lieu de récolte, l'année de récolte, la saison, les conditions climatiques, etc.

Dans cet exemple, on obtient plus particulièrement un extrait aqueux titrant 17.9 g/kg d'extrait de poids sec, 2.1 g/kg de fragments protéiques, 11.4 g/kg de sucres, 200 mg/kg de composés phénoliques et 54 mg/kg d'ARN de petit poids moléculaire d'une longueur d'au maximum 150 nucléotides.

L'extrait est alors dilué dans un mélange d'eau, 30% de glycérol et 1,5 % de phénoxyéthanol, ce qui permet d'obtenir un extrait final à 10 g/Kg de matière séche.

L'analyse physico-chimique montre qu'après dilution cet extrait présente une concentration en fragments protéiques de 1.0 g/kg, en sucres de 5.8 g/kg, en composés phénoliques de 100 mg/kg, et de 30 mg/kg d'ARN de petit poids moléculaire d'une longueur d'au maximum 150 nucléotides.

### Exemple 16 : Préparation d'un extrait de fruit de la passion (Passiflora alata) de la famille des Passifloraceae enrichi en petits ARN

Un extrait aqueux enrichi en ARN de petit poids moléculaire (d'une longueur d'au maximum 150 nucléotides) est obtenu à partir de fruit de la passion (*Passiflora alata*) de la famille des passifloraceae.

Dans une première étape, 5% de poudre de fuit sont mélangés dans de l'eau distillée, soit par exemple 50 g de poudre de fruit complété à 1 kg d'eau distillée, puis la solution est mise à agiter pendant 5 minutes puis de l'EDTA tétrasodique à une concentration finale de 10 mM soit pour 1kg, 3,8 g est ajouté. Le pH à cette étape est compris entre 10,5 et 11, pH optimal pour enrichir l'extrait en petits ARN.

Ce mélange est ensuite mis sous agitation pendant 1 heure à 50°C. Même si la température à ce stade peut varier de 25°C à 80°C et le temps d'agitation de 30 minutes à 1h, pour cette espèce une température de 50°C pendant 60 minutes s'avère être les conditions qui permettent d'obtenir les meilleurs résultats en terme d'enrichissement de l'extrait aqueux final en ARN de petit poids moléculaire.

A l'issue de cette étape, Le mélange est centrifugé, 10 min à 4000 g, pour ôter la matière solide.

Puis des filtrations séquentielles sont alors réalisées sur des filtres de porosité décroissante de taille comprise entre 20 et 50 µm puis 7 et 20 µm jusqu'à 2-4 µm, afin de clarifier l'extrait végétal.

A cette étape, le pH est contrôlé, afin de le placer si besoin entre 6 et 6,5 et préserver les petits ARN de l'extrait qui sont sensibles à un pH acide.

Les filtrations sont poursuivies jusqu'à une porosité de 0,3 à 0,5 µm.

De manière générale, on obtient un extrait aqueux de couleur ambré titrant de 10 à 30 g/kg d'extrait de poids sec, 0,5 à 5 g/kg de fragments protéiques, 2 à 15 g/kg de sucres, 100 à 1500 mg/kg de composés phénoliques et 10 à 100 mg/kg d'ARN de petit poids moléculaire d'une longueur d'au maximum 150 nucléotides.

L'extrait peut ensuite être dilué ou conservé grâce à l'ajout d'un solvant physiologiquement acceptable tel que du glycérol à 30%.

Dans cet exemple, on obtient après ajout de 30% de solvant un extrait aqueux titrant à 15 g/kg d'extrait de poids sec, 2.3 g/kg de fragments protéiques, 3.0 g/kg de sucres, 200 mg/kg de composés phénoliques et 35 mg/kg d'ARN de petit poids moléculaire d'une longueur d'au maximum 150 nucléotides.

Selon un troisième aspect de l'invention, les extraits aqueux enrichis en petits ARN obtenus selon l'invention sont avantageusement utilisés dans la préparation de compositions cosmétiques comprenant, à titre d'agent actif anti-âge, une quantité efficace d'un tel extrait de petits ARN selon l'invention, et un milieu physiologiquement acceptable.

Par quantité efficace, on désigne la quantité minimum d'extrait selon l'invention qui est nécessaire pour obtenir l'activité de l'extrait, en particulier cosmétique et plus particulièrement contre les signes du vieillissement cutané ou pour l'amélioration de l'hydratation de la peau, sans que cette quantité soit toxique.

Avantageusement, l'extrait de petits ARN selon l'invention est utilisé sous sa forme diluée, d'un poids sec compris entre 5 à 35 g/kg.

Avantageusement, l'extrait de petits ARN selon l'invention est présent dans la composition à une concentration de 0,1 à 5 %, préférentiellement à une concentration de 1 à 5% en poids par rapport au poids total de la composition.

Un milieu physiologiquement acceptable désigne un véhicule adapté pour une mise en contact avec les couches externes de la peau ou des muqueuses, sans toxicité, irritation, réponse allergique indue et similaire ou réaction d'intolérance, et proportionné à un rapport avantage/risque raisonnable.

La composition utilisable selon l'invention pourra être appliquée par toute voie appropriée, notamment orale, ou topique externe, et la formulation des compositions sera adaptée par l'homme du métier.

Préférentiellement, les compositions selon l'invention se présentent sous une forme adaptée à l'application par voie topique. Ces compositions doivent donc contenir un milieu physiologiquement acceptable, c'est-à-dire compatible avec la peau et les phanères, sans risque d'inconfort lors de leur application et couvrent toutes les formes cosmétiques adaptées.

Par application topique, on désigne le fait d'appliquer ou d'étaler l'extrait aqueux enrichi en petits ARN selon l'invention, et plus particulièrement une composition le contenant, à la surface de la peau ou d'une muqueuse.

La peau désigne plus particulièrement la peau du visage, notamment le contour des yeux et la bouche, le nez, le front, le cou, les mains, ainsi que la peau de l'ensemble du reste du corps.

Les compositions pour la mise en œuvre de l'invention pourront notamment se présenter sous forme d'une solution aqueuse, hydro-alcoolique ou huileuse, d'une émulsion huile-dans-eau, eau-dans-huile ou émulsions multiples ; elles peuvent aussi se présenter sous forme de suspensions, ou encore poudres, adaptées à une application sur la peau, les muqueuses, les lèvres et/ou les cheveux.

Ces compositions peuvent être plus ou moins fluides et avoir également l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

A titre de milieu physiologiquement acceptable communément utilisé dans le domaine d'application envisagé, on peut citer par exemple des adjuvants nécessaires à la formulation, tels que des solvants, des épaississants, des diluants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20% du poids total de la composition. Lorsque la composition selon l'invention est une émulsion, la phase grasse peut représenter de 5 à 80% en poids et de préférence de 5 à 50% en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30% en poids par rapport au poids total de la composition.

Selon un autre mode de réalisation avantageux de l'invention, l'extrait aqueux enrichi en petits ARN selon l'invention peut être encapsulé ou inclus dans un vecteur cosmétique tels que les liposomes ou toute autre nanocapsule ou microcapsule utilisée dans le domaine de la cosmétique ou adsorbé sur des polymères organiques poudreux, des supports minéraux comme les talcs et bentonites.

Avantageusement, la composition selon l'invention peut comprendre, outre l'agent actif selon l'invention, au moins un autre agent actif présentant des effets cosmétiques similaires et/ou complémentaires à ceux de l'invention. Selon l'invention, cet agent actif est défini comme un « agent actif additionnel ».

Par exemple, le ou les agents actifs additionnels peuvent être choisis parmi : les agents anti-âge, raffermissants, éclaircissants, hydratants, drainants, favorisant la microcirculation, exfoliants, desquamants, stimulant la matrice extracellulaire, activant le métabolisme énergétique, antibactériens, antifongiques, apaisants, anti-radicalaires, anti-UV, anti-acné, anti-inflammatoires, anesthésiques, procurant une sensation de chaleur, procurant une sensation de fraicheur, amincissants.

De tels agents actifs additionnels peuvent être choisis dans les groupes comprenant :
- la vitamine A et notamment l'acide rétinoïque, le rétinol, le rétinol proprionate, le rétinol palmitate ;
- la vitamine B3 et plus particulièrement le niacinamide, le nicotinate de tocophérol ;
- la vitamine B5, la vitamine B6, la vitamine B12, le panthénol ;
- la vitamine C, notamment l'acide ascorbique, l'ascorbyl glucoside, l'ascorbyl tétrapalmitate, magnésium et sodium ascorbyl phosphate ;
- les vitamines E, F, H, K, PP, le coenzyme Q10 ;
- les inhibiteurs de métalloprotéinase, ou un activateur des TIMP ;
- la DHEA, ses précurseurs et dérivés ;
- les acides aminés tels que l'arginine, ornithine, hydroxyproline, hydroxyproline dipalmitate, palmitoylglycine, hydroxylysine, méthionine et ses dérivés, composés acides aminés N-acylés ;
- les peptides naturels ou de synthèse, incluant les di-, tri-, tetra-, penta- et hexapeptides et leurs dérivés lipophiles, isomères et complexés avec d'autres espèces telles qu'un ion métal (par exemple cuivre, zinc, manganèse, magnésium, et autres). A titre d'exemples, on peut citer les peptides commercialement connus sous le nom de MATRIXYL®, ARGIRELINE®, CHRONOGEN™, LAMINIXYL IS™, PEPTIDE Q10™, COLLAXYL™ (brevet FR2827170, ASHLAND®), PEPTIDE VINCI 01™ (brevet FR2837098, ASHLAND®), PEPTIDE VINCI 02™ (brevet FR2841781, ASHLAND®), ATPeptide™ (brevet FR2846883, ASHLAND®) ou encore le peptide de synthèse de séquence Arg-Gly-Ser-NH₂, commercialisé sous le nom ATPeptide™ par ASHLAND® ;
- l'extrait d*'Artémia salina,* commercialisé sous le nom GP4G™ (FR2817748, ASHLAND®) ;
- les extraits peptidiques végétaux tels que les extraits de lin (Lipigénine™, brevet FR2956818, ASHLAND®), les extraits de soja, d'épeautre, de vigne, de colza, de lin, de riz, de maïs, de pois ;
- les extraits de levures, par exemple le Dynagen™, (brevet FR2951946, ASHLAND®) ou l'Actopontine™ (brevet FR2944526, ASHLAND®) ;
- l'acide déhydroacétique (DHA) ;
- les phystostérols d'origine synthétique ou naturelle ;
- l'acide salicylique et ses dérivés, les alpha- et bêta-hydroxyacides, les silanols ;
- les sucres aminés, glucosamine, D-glucosamine, N-acetyl glucosamine, N-acetyl-D-glucosamine, mannosamine, N-acetyl mannosamine, galactosamine, N-acetyl galactosamine ;
- les extraits de polyphénols, isoflavones, flavonoïdes, tels que les extraits de raisin, les extraits de pin, les extraits d'olive ;
- les lipides tels que les céramides ou les phospholipides, les huiles d'origine animale, telles que le squalène ou le squalane ; les huiles végétales, telles que l'huile d'amande douce, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, le beurre de karité ;
- tous écrans UV et filtres solaires ;
- l'AMP cyclique et ses dérivés, les agents activateurs de l'enzyme adénylate cyclase et les agents inhibiteurs de l'enzyme phosphodiestérase, l'extrait de *Centella asiatica,* l'asiaticoside et l'acide asiatique, les méthyls xanthines, la théine, la caféine et ses dérivés, la théophylline, la théobromine, la forskoline, l'esculine et l'esculoside, les inhibiteurs d'ACE, le peptide Val-Trp, l'inhibiteurs du neuropeptide Y, l'enkephaline, l'extrait de *Ginkgo biloba,* l'extrait de *dioscorea,* la rutine, l'extrait de yerba mate, l'extrait de guarana, les oligosaccharides, les polysaccharides, la carnitine, l'extrait de lierre, l'extrait de fucus, l'extrait hydrolysé de *Prunella vulgaris,* l'extrait hydrolysé de *Celosia cristata,* l'extrait d'*Anogeissus leiocarpus,* l'extrait de feuilles de *Manihot utilissima,* la palmitoylcarnitine, la carnosine, la taurine, l'extrait de sureau, les extraits d'algue tel que l'extrait de *Palmaria Palmata.*

A titre d'illustration, il est mentionné ci-après des exemples de formulations d'une composition cosmétique contenant un extrait aqueux enrichi en petits ARN d'une longueur d'au maximum 150 nucléotides obtenu selon l'invention :

### Exemple 17 : Baume pour le contour des yeux

| Ingrédients (Nom de marque) | INCI | % w/w |
|---|---|---|
| Phase A | | |
| Eau purifiée | Aqua | Qsp 100 |
| EDTA tétrasodique | Tetrasodium EDTA | 0,01 |

| Phase B | | |
|---|---|---|
| RapiThix™ A-100 polymer | Sodium Polyacrylate | 1,80 |

| Phase C | | |
|---|---|---|
| Cegesoft VP | Vegetable oil (and) Hydrogenated | 3,00 |
| | vegetable oil (and) *Euphorbia* | |
| | *Cerifera* (*Candelilla*) Wax | |
| Si-Tec™ GF 3096 silicone | Dimethicone (and) Dimethiconol | 10,00 |

| Phase D | | |
|---|---|---|
| DC 9701 Cosmetic Powder | Dimethicone/Vinyl Dimethicone Crosspolymer (and) Silica | 1,00 |

| Phase E | | |
|---|---|---|
| Optiphen™ preservative | Phenoxyethanol (and) Caprylyl Glycol | 0,50 |

| Phase F | | |
|---|---|---|
| Extrait selon l'exemple 5 | Water/Aqua (and) Glycerin (and) Hydrolyzed *Hibiscus esculentus* extract | 1,00 |
| Zemea® | Propanediol | 5,00 |
| Timiron Splendid Violet | CI 77891 (Titanium Dioxide) (and) Mica (and) Silica | 1,00 |

### Procédé de préparation:

1. Homogénéiser la phase A dans le récipient principal jusqu'à ce qu'elle soit claire ;
2. A 25°C, saupoudrer dans la Phase B et homogénéiser pendant 10 minutes jusqu'à homogénéité ;
3. A 25°C, préparer la phase C dans un bécher à part, mélanger jusqu'à homogénéité. Saupoudrer dans la phase D et bien mélanger jusqu'à homogénéité ;
4. A 25°C, ajouter la phase C + D dans le récipient principal et mélanger jusqu'à homogénéité ;
5. À 25°C, ajouter la phase E dans le récipient principal et mélanger jusqu'à homogénéité ;
6. A 25°C, prémélanger la phase F, l'ajouter dans le récipient principal et mélanger jusqu'à homogénéité ;
7. Arrêter à 25°C.

La composition se présente ainsi sous forme d'un gel crème nacré violet, avec un pH compris entre 5,70 et 6,20 et une viscosité (D0) de 80000 - 130000 cps (Brookfield RVT/Spindle C/5 RPM/1 minute/25°C).

### Exemple 18 : Crème riche

| Ingrédients (Nom de marque) | INCI | % w/w |
|---|---|---|
| Phase A | | |
| Eau purifiée | Aqua | Qsp 100 |
| Optiphen™ Plus preservative | Phenoxyethanol (and) Caprylyl Glycol (and) Sorbic Acid | 1,50 |

| Phase B | | |
|---|---|---|
| Stabileze™ QM polymer | PVM/MA Decadiene Crosspolymer | 0,15 |

| Phase C | | |
|---|---|---|
| ProLipid™ 141 lamellar gel | Glyceryl Stearate (and) Behenyl Alcohol (and) Palmitic Acid (and) Stearic Acid (and) Lecithin (and) Lauryl Alcohol (and) Myristyl Alcohol (and) Cetyl Alcohol | 5,00 |
| Ceraphyl™ 494 ester | Isocetyl Stearate | 4,00 |
| Ceraphyl™ SLK ester | Isodecyl Neopentanoate | 4,00 |
| DC 580 Wax | Stearoxytrimethylsilane (and) Stearyl Alcohol | 2,00 |
| Emulsynt™ GDL ester | Glyceryl Dilaurate | 3,00 |

| Phase D | | |
|---|---|---|
| Gransil DM-5 | Dimethicone (and) Polysilicone-11 | 3,00 |

| Phase E | | |
|---|---|---|
| Hydroxyde de sodium | Sodium Hydroxide | 0,04 |
| Eau purifiée | Aqua | 0,50 |

| Phase F | | |
|---|---|---|
| PF Bois Précieux | Parfum/Fragrance | 0,30 |
| Unipure* Red LC 381 ADT-C | CI 77491 (Iron oxides) (and) Isopropyl Titanium Triisostearate (and) Bis-Hydroxyethoxypropyl Dimethicone (and) PEG-2-Soyamine (and) Isophorone Diisocyanate | 0,03 |

| Phase G | | |
|---|---|---|
| Extrait selon l'exemple 3 | Water/Aqua (and) Glycerin (and) *Lens esculenta* seed extract | 3,00 |
| Ronaflair Balance Gold | CI 77891 (Titanium Dioxide) (and) Mica (and) Tin Oxide | 0,30 |
| Covabead Velvet 10 | Polymethyl Methacrylate | 1,00 |
| Ronaflair Balance Red | CI 77891 (Titanium Dioxide) (and) Mica (and) Tin Oxide | 1,20 |

| Phase H | | |
|---|---|---|
| Eau purifiée | Aqua | 15,00 |
| Natrosol™ Plus 330 CS | Cetyl Hydroxyethylcellulose | 0,50 |

### Procédé de préparation:

1. Homogénéiser la phase A dans le récipient principal et commencer à chauffer à 75-80°C ;
2. A 30°C, saupoudrer dans la Phase B et homogénéiser tout en chauffant ;
3. Dans un bécher à part, préparer la phase C, chauffer à 75-80°C jusqu'à homogénéité ;
4. A 75°C, ajouter la phase C dans le récipient principal et homogénéiser pendant 10 minutes ;
5. Laisser refroidir la température et ajouter la phase D à 65°C. Bien mélanger pour homogénéiser pendant 10 minutes ;
6. Prémélanger la phase E avant de l'ajouter dans le récipient principal ;
7. Ajouter la phase E à 60°C. Bien mélanger pour homogénéiser pendant 10 minutes ;
8. A 35°C, prémélanger la phase F avant de l'ajouter et de bien mélanger ;
9. Prémélanger la phase G avant de l'ajouter dans le récipient principal ;
10. Ajouter la phase G à 35°C. Bien mélanger pour homogénéiser ;
11. Dans un bêcher à part, préparer la phase H: saupoudrer Natrosol™ dans l'eau à température ambiante et homogénéiser tout en chauffant à 60°C ;
12. Ajouter la phase H à 30°C. Bien mélanger pour homogénéiser ;
13. Arrêter à 25°C.

La composition se présente ainsi sous forme d'une crème beurre rose, avec un pH compris entre 4,90 et 5,40 et une viscosité (D0) de 160000 - 210000 cps (Brookfield RVT/Spindle D/5 RPM/1 minute/25°C).

### Exemple 19 : Sérum visage

| Ingrédients (Nom de marque) | INCI | % w/w |
|---|---|---|
| Phase A | | |
| Eau purifiée | Aqua | Qsp 100 |
| Propylène Glycol | Propylene Glycol | 35,10 |
| SD Alcohol 40B Absolute | Alcohol | 10,00 |
| Butylène Glycol | Butylene Glycol | 5,00 |
| EDTA disodique | Disodium EDTA | 0,10 |

| Phase B | | |
|---|---|---|
| Flexithix™ polymer | PVP | 2,00 |

| Phase C | | |
|---|---|---|
| Gomme xanthane | Xanthan Gum | 0,25 |

| Phase D | | |
|---|---|---|
| Rapithix™ A-60 polymer | Sodium Polyacrylate (and) Hydrogenated Polydecene (and) Trideceth-6 | 1,00 |
| Rokonsal™/LiquaPar™ MEP preservative | Phenoxyethanol (and) Methylparaben (and) Ethylparaben (and) Propylparaben | 0,70 |

| Phase E | | |
|---|---|---|
| Extrait selon l'exemple 7 | Water/Aqua (and) Glycerin (and) Hydrolyzed *Adansonia digitata* extract | 5,00 |

| Phase F | | |
|---|---|---|
| Cyclopentasiloxane | Cyclopentasiloxane | 6,00 |
| DM 350 | Dimethicone | 3,00 |
| Gransil* DMCM-5 | Dimethicone (and) Cyclopentasiloxane (and) Polysilicone-11 | 1,50 |
| KSP* 100 | Vinyl Dimethicone/Methicone Silsesquioxane Crosspolymer | 1,00 |

| Phase G | | |
|---|---|---|
| Unicert* Yellow 08006-J | Water/Aqua (and) CI 15985 (Yellow 6) | 0,60 |

### Procédé de préparation :

1. Dans un bécher à température ambiante, peser les ingrédients de la phase A et mélanger. Saupoudrer la phase B et homogénéiser ;
2. A température ambiante, saupoudrer dans la phase C et continuer à homogénéiser l'ensemble ;
3. A température ambiante, ajouter la phase D à la phase ABC et continuer à homogénéiser ;
4. A température ambiante, ajouter la phase E et homogénéiser ;
5. A température ambiante, ajouter la phase F et homogénéiser l'ensemble ;
6. A température ambiante, ajouter la phase G et mélanger jusqu'à homogénéité ;
7. Arrêter à 25°C.

La composition se présente ainsi sous forme d'un gel lisse, translucide, jaune crème, avec un pH compris entre 6,30 et 7,10 et une viscosité (D0) de 10000 - 15000 cps (Brookfield RVT/Spindle B/5 RPM/1 minute/25°C).

### Exemple 20 : Masque anti-âge

| Ingrédients (Nom de marque) | INCI | % w/w |
|---|---|---|
| Phase A | | |
| Eau purifiée | Aqua | Qsp 100 |
| EDTA tétrasodique | Tetrasodium EDTA | 0,05 |

| Phase B | | |
|---|---|---|
| N-Hance™ HP40S guar | Hydroxypropyl Guar | 0,10 |

| Phase C | | |
|---|---|---|
| Lubrajel™ DV Free hydrogel | Glycerin (and) Glyceryl Acrylate/Acrylic Acid Copolymer | 6,00 |

| Phase D | | |
|---|---|---|
| Si-Tec™ GF 3096 silicone | Dimethicone (and) Dimethiconol | 12,00 |
| RapiThix™ A-60 polymer | Sodium Polyacrylate (and) Hydrogenated Polydecene (and) Trideceth-6 | 2,40 |

| Phase E | | |
|---|---|---|
| Optiphen™ Plus preservative | Phenoxyethanol (and) Caprylyl Glycol (and) Sorbic Acid | 1,50 |

| Phase F | | |
|---|---|---|
| Surfin* 96 | Alcohol Denat. | 3,50 |
| PF Cucumber & Aloe | Parfum/Fragrance | 0,50 |

| Phase G | | |
|---|---|---|
| Extrait selon l'exemple 10 | Water/Aqua (and) Glycerin (and) Hydrolyzed *Cucurbita pepo* seedcake extract | 1,00 |
| Achromaxyl™ ISR biofunctional | Water/Aqua (and) Glycerin (and) Hydrolyzed Brassica Napus Seedcake Extract | 3,00 |
| Xirona Carribean Blue | Mica (and) CI 77891 (Titanium Dioxide) (and) Silica (and) Tin Oxide | 1,00 |

### Procédé de préparation:

1. A 25°C, homogénéiser la phase A dans le récipient principal ;
2. A 25°C, saupoudrer dans la phase B et bien mélanger jusqu'à homogénéité ;
3. A 25°C, ajouter la phase C et bien mélanger jusqu'à homogénéité ;
4. Prémélanger la phase D dans un bêcher à part et ajouter dans le récipient principal à 25°C ;
5. A 25°C, ajouter la phase E dans le récipient principal et bien mélanger ;
6. Prémélanger la phase F et l'ajouter lentement. Bien mélanger bien jusqu'à homogénéité ;
7. Prémélanger la phase G dans un bêcher à part et ajouter dans le récipient principal jusqu'à homogénéité ;
8. Arrêter à 25°C.

La composition se présente ainsi sous forme d'un gel crème avec des effets vert scintillant, avec un pH compris entre 5,30 et 5,80 et une viscosité (D0) de 70000 - 100000 cps (Brookfield RVT/Spindle C/5 RPM/1 minute/25°C).

### Exemple 21 : Sérum

| Ingrédients (Nom de marque) | INCI | % w/w |
|---|---|---|
| Phase A | | |
| Eau déminéralisée | Aqua | 87,40 |
| Sodium Hyaluronate | Sodium Hyaluronate | 0,20 |
| RapiThix™ A-60 polymer | Sodium Polyacrylate (and) Hydrogenated Polydecene (and) Trideceth-6 | 0,40 |
| Lubrajel™ DV hydrogel | Glycerin (and) Glyceryl Acrylate/Acrylic Acid Copolymer (and) Propylene Glycol | 6,00 |
| Lubrajel™ Oil hydrogel | Glycerin (and) Glyceryl Acrylate/Acrylic Acid Copolymer (and) Propylene Glycol (and) PVM/MA Copolymer | 1,00 |
| Wacker-Belsil* DM 100 | Dimethicone | 2,00 |
| Cyclopentasiloxane NF | Cyclopentasiloxane | 0,50 |
| Extrait selon l'exemple 11 | Water/Aqua (and) Glycerin (and) *Chenopodium quinoa* seed extract | 1,00 |
| Optiphen™ preservative | Phenoxyethanol (and) Caprylyl Glycol | 1,50 |

### Procédé de préparation:

1. Ajouter de l'eau dans le récipient principal et de commencer le mélange avec une pale d'hélice hi-lo ;
2. Ajouter le reste des ingrédients, un après l'autre tout en mélangeant entre chaque addition.

La composition se présente ainsi sous forme d'un sérum lisse, semi-opaque, avec un pH compris entre 5,75 et 6,25 et une viscosité (D0) de 1,100 - 1,400 cps (Brookfield RVT/spindle 3/20 rpm/25° C/1 minute).

Selon un quatrième aspect, l'invention concerne l'utilisation cosmétique de la composition selon l'invention pour lutter contre les signes du vieillissement cutané.

Par « signes du vieillissement cutané » on entend toutes modifications de l'aspect extérieur de la peau dues au vieillissement comme, par exemple, les rides et ridules, les crevasses, les poches sous les yeux, les cernes, le flétrissement, la perte d'élasticité, de fermeté et/ou de tonus de la peau, mais également toutes modifications internes de la peau qui ne se traduisent pas systématiquement par un aspect extérieur modifié comme, par exemple, l'amincissement de la peau, ou toutes dégradations internes de la peau consécutives à des stress environnementaux tels que la pollution et les rayonnements UV.

L'étude de l'expression des collagènes de la peau est un moyen de juger de l'effet anti-âge de l'invention. En effet, le collagène synthétisé par les fibroblastes de peau a un important rôle biologique. Il est responsable de la cohésion des tissus tels que la peau et confère des propriétés de résistance et de souplesse à la peau.

L'invention concerne également l'utilisation cosmétique de la composition selon l'invention pour améliorer l'hydratation de la peau.

On entend par amélioration de l'hydratation cutanée, toutes améliorations des modifications de l'aspect extérieur de la peau dues à la déshydratation comme, par exemple, la sécheresse, les tiraillements et l'inconfort.

L'étude de l'expression de l'acide hyaluronique ainsi que de l'enzyme impliquée dans la synthèse de l'acide hyaluronique est un moyen de juger de l'effet hydratant de l'invention. En effet, l'acide hyaluronique est un composant majoritaire de la matrice extracellulaire du derme, également présent dans l'épiderme, et est impliqué dans l'hydratation cutanée.

A ce titre, l'invention est illustrée ci-après, par les différents résultats de tests réalisés.

A cet effet, des résultats analogues (non représentés) à ceux représentés dans les exemples 22 à 25 ci-après ont été obtenus avec d'autres extraits aqueux enrichi en petits ARN d'une longueur d'au maximum 150 nucléotides selon l'invention obtenus à partir de matière végétale, et plus particulièrement à partir de *Chenopodium quinoa* (quinoa), *Lens esculenta* (lentille), *Oryza sativa* (germes de riz), ou encore *Cucurbita pepo* (courge).

### Exemple 22 : Evaluation des effets des extraits de gombo (Hibiscus esculentus) selon les exemples 5, 6 et 9 sur la matrice extracellulaire du derme par l'étude des collagènes I et III

Le but de cette étude est de comparer les effets sur la matrice extracellulaire du derme de trois extraits de gombo (*Hibiscus esculentus*). Le premier extrait enrichi en ARN de petit poids moléculaire, obtenu selon l'exemple 5, le deuxième extrait obtenu selon l'exemple 6 (absence de traitement par EDTA) et le dernier extrait obtenu après précipitation selon l'exemple 9.

Le but de cette étude est d'évaluer les effets de ces trois extraits d'*Hibiscus esculentus* sur l'expression des protéines collagène I et III impliquées dans la structure de la matrice extracellulaire. Le collagène est très important pour le maintien de l'élasticité et de la fermeté de la peau.

### Protocole:

Des biopsies de peau humaine de 6 mm de diamètre sont maintenues en culture *ex vivo* en présence d'un milieu spécifique (DMEM 1 g/L, HAMF12, SVF et antibiotiques) sur des inserts déposés dans des plaques 6 puits. Les biopsies sont cultivées pendant 48h et reçoivent 2 applications par jour d'un extrait de gombo (*Hibiscus esculentus*) selon les exemples 5, 6 et 9 dilué au 1/100eme ou au 3/100eme dans du PBS, soit respectivement à la concentration finale de 1% et de 3% volume/volume. La condition contrôle est réalisée à l'aide de PBS 1X. Les applications sont réalisées sous forme d'une goutte d'environ 20 µl déposée à la surface de la biopsie. Les biopsies sont ensuite fixées dans le formaldéhyde puis incluses dans la paraffine. Des coupes de peau de 4 µm d'épaisseur sont ensuite réalisées. Les marquages des collagènes I et III sont effectués après démasquage des sites spécifiques par une incubation au micro-onde puis par un traitement à la trypsine. Les immunomarquages sont réalisés à l'aide d'un anticorps polyclonal de lapin spécifique du collagène I (Rockland, Réf. 600-401-103-0.5), d'un anticorps polyclonal de lapin spécifique du collagène III (Rockland, Réf. 600-401-105-0,5), puis d'un anticorps secondaire anti-lapin couplé à un fluorochrome (Invitrogen, Réf. A21206). Les biopsies sont alors examinées au microscope à Epi-fluorescence (Zeiss Axiovert 200M microscope). Une quantification de la fluorescence, à l'aide du logiciel Volocity® image analysis software (PerkinElmer, Inc.) a été réalisée à partir des photographies obtenues.

### Résultats:

Les traitements avec l'extrait de gombo (*Hibiscus esculentus*) enrichi en petits ARN, obtenu selon l'exemple 5, à 1% et 3% permettent d'observer une augmentation significative de l'expression des collagènes I et III comparé à la condition contrôle traitée avec du PBS 1X et comparé aux traitements avec les extraits de gombo (*Hibiscus esculentus*) obtenus selon les exemples 6 (absence de traitement par EDTA) et 9 (obtenu après précipitation), pour l'étude *ex vivo.*

| | Expression du collagène I (%) | Expression du collagène III (%) |
|---|---|---|
| Non traité | 100 | 100 |
| Extrait *d'Hibiscus esculentus* selon l'exemple 5 à 1% | 116, 3 | 136,2 |
| Extrait d'*Hibiscus esculentus* selon l'exemple 5 à 3% | 112,7 | 179,2 |
| Extrait *d'Hibiscus esculentus* selon l'exemple 6 à 1% | 101,6 | 115,6 |
| Extrait *d'Hibiscus esculentus* selon l'exemple 6 à 3% | 102, 9 | 100 |
| Extrait *d'Hibiscus esculentus* selon l'exemple 9 à 1% | 114,5 | 118, 9 |
| Extrait d'*Hibiscus esculentus* selon l'exemple 9 à 3% | 100,5 | 116, 8 |

### Conclusions:

L'extrait de gombo *(Hibiscus esculentus)* enrichi en petits ARN (exemple 5) à 1 et 3% stimule davantage l'expression des collagènes I et III dans la peau ex *vivo* humaine en comparaison aux deux extraits *d'Hibiscus esculentus* non enrichis en petits ARN (exemples 6 et 9) à 1% et 3%.

### Exemple 23 : Evaluation des effets des extraits de baobab (Adansiona digitata) selon les exemples 7, 8 et 9 sur la matrice extracellulaire du derme par l'étude des collagènes I et III

Le but de cette étude est de comparer les effets sur la matrice extracellulaire du derme de trois extraits de baobab *(Adansiona digitata).* Le premier extrait enrichi en ARN de petit poids moléculaire, obtenu selon l'exemple 7, le deuxième extrait obtenu selon l'exemple 8 (absence de traitement par EDTA) et le dernier extrait obtenu après précipitation selon l'exemple 9.

Le but de cette étude est d'évaluer les effets de ces trois extraits de baobab *(Adansiona digitata)* sur l'expression des protéines collagène I et III impliquées dans la structure de la matrice extracellulaire. Le collagène est très important pour le maintien de l'élasticité et de la fermeté de la peau.

### Protocole:

Des biopsies de peau humaine de 6 mm de diamètre sont maintenues en culture ex *vivo* en présence d'un milieu spécifique (DMEM 1 g/L, HAMF12, SVF et antibiotiques) sur des inserts déposés dans des plaques 6 puits. Les biopsies sont cultivées pendant 48h et reçoivent 2 applications par jour d'un extrait de baobab *(Adansiona digitata)* selon les exemples 7, 8 et 9 dilué au 1/100eme ou au 3/100eme dans du PBS, soit respectivement à la concentration finale de 1% et de 3% volume/volume. La condition contrôle est réalisée à l'aide de PBS 1X. Les applications sont réalisées sous forme d'une goutte d'environ 20 µl déposée à la surface de la biopsie. Les biopsies sont ensuite fixées dans le formaldéhyde puis incluses dans la paraffine. Des coupes de peau de 4 µm d'épaisseur sont ensuite réalisées. Les marquages des collagènes I et III sont effectués après démasquage des sites spécifiques par une incubation au micro-onde puis par un traitement à la trypsine. Les immunomarquages sont réalisés à l'aide d'un anticorps polyclonal de lapin spécifique du collagène I (Rockland, Réf. 600-401-103-0.5), d'un anticorps polyclonal de lapin spécifique du collagène III (Rockland, Réf. 600-401-105-0.5), puis d'un anticorps secondaire anti-lapin couplé à un fluorochrome (Invitrogen, Réf. A21206). Les biopsies sont alors examinées au microscope à Epi-fluorescence (Zeiss Axiovert 200M microscope). Une quantification de la fluorescence, à l'aide du logiciel Volocity® image analysis software (PerkinElmer, Inc.) a été réalisée à partir des photographies obtenues.

### Résultats:

Le traitement avec l'extrait de baobab *(Adansiona digitata)* enrichi en petits ARN, obtenu selon l'exemple 7, à 1% permet d'observer une augmentation significative de l'expression du collagène I comparé à la condition contrôle traitée avec du PBS 1X et comparé aux traitements à 1% avec les extraits *d'Adansiona digitata* obtenus selon les exemples 8 (absence de traitement par EDTA) et 9 (obtenu après précipitation), pour l'étude *ex vivo.*

Le traitement avec l'extrait de baobab *(Adansiona digitata)* enrichi en petits ARN, obtenu selon l'exemple 7, à 3% permet d'observer une augmentation significative de l'expression des collagènes I et III comparé à la condition contrôle traitée avec du PBS 1X et comparé aux traitements à 3% avec les extraits de baobab *(Adansiona digitata)* obtenus selon les exemples 8 (absence de traitement par EDTA) et 9 (obtenu après précipitation), pour l'étude ex *vivo.*

| | Expression du collagène I (%) | Expression du collagène III (%) |
|---|---|---|
| Non traité | 100 | 100 |
| Extrait *d'Adansiona digitata* selon l'exemple 7 à 1% | 130 | - |
| Extrait *d'Adansiona digitata* selon l'exemple 7 à 3% | 134,4 | 149 |
| Extrait *d'Adansiona digitata* selon l'exemple 8 à 1% | 103,2 | - |
| Extrait *d'Adansiona digitata* selon l'exemple 8 à 3% | 107,2 | 107,4 |
| Extrait *d'Adansiona digitata* selon l'exemple 9 à 1% | 100 | - |
| Extrait *d'Adansiona digitata* selon l'exemple 9 à 3% | 100 | 110,8 |

### Conclusions:

L'extrait de baobab *(Adansiona digitata)* enrichi en petits ARN (exemple 7) à 1% stimule davantage l'expression du collagène I dans la peau ex *vivo humaine* en comparaison aux extraits *d'Adansiona digitata* non enrichis en petits ARN (exemples 8 et 9) à 1%.

L'extrait de baobab *(Adansiona digitata)* enrichi en petits ARN (exemple 7) à 3% stimule davantage l'expression des collagènes I et III dans la peau ex *vivo* humaine en comparaison aux extraits *d'Adansiona digitata* non enrichis en petits ARN (exemples 8 et 9) à 3%.

### Exemple 24 : Evaluation des effets des extraits de baobab (Adansiona digitata) selon les exemples 7, 8 et 9 sur la synthèse de l'acide hyaluronique du derme par l'étude de l'hyaluronane synthase 2 (HAS2)

Le but de cette étude est de comparer les effets de trois extraits de baobab *(Adansiona digitata)* sur l'expression de l'HAS2, enzyme impliquée dans la synthèse de l'acide hyaluronique. Le premier extrait enrichi en ARN de petit poids moléculaire, est obtenu selon l'exemple 7, le deuxième extrait obtenu selon l'exemple 8 (absence de traitement par EDTA) et le dernier extrait obtenu après précipitation selon l'exemple 9.

L'acide hyaluronique est un composant majoritaire de la matrice extracellulaire du derme, impliqué dans l'hydratation de la peau. Lors du vieillissement, son renouvellement est perturbé, ainsi que l'expression de son enzyme de synthèse HAS2 (Rock et *al.,* 2014).

### Protocole:

Des fibroblastes humains sont mis en culture dans un milieu spécifique et maintenus en culture en traitement long (pendant plus de 32 passages). A chaque passage, une partie des cellules est congelée. Puis, deux passages opposés sont choisis : passage 8 (passage jeune) et passage 32 (passage sénescent). Après décongélation, les fibroblastes sénescents ou non sont traités avec les extraits de baobab (*Adansiona digitata*) selon les exemples 7, 8 et 9 dilués au 1/100eme dans du milieu de culture, soit à la concentration finale de 1% volume/volume, 48 heures (2 applications par jour). L'évaluation de l'expression de HAS2 sur les fibroblastes sénescents ou non, traités ou non avec les trois extraits *d'Adansiona digitata* selon les exemples 7, 8 et 9 à 1% est observée par immunomarquage.

Pour se faire, les cellules sont rincées et fixées au méthanol froid pendant 5 minutes. Après saturation des sites non spécifiques par de l'albumine de sérum bovin à 1% pendant 15 min, les cellules sont incubées avec une solution d'anticorps monoclonal de souris spécifique de l'HAS2 (Thermo Fisher, Réf. MAS-17087), puis d'une solution d'anticorps secondaire anti-souris couplé à un fluorochrome (Invitrogen, Réf. A21202). Les cellules sont alors examinées au microscope à Epi-fluorescence (Zeiss Axiovert 200M microscope). Une quantification de la fluorescence, à l'aide du logiciel Volocity® image analysis software (PerkinElmer, Inc.) a été réalisée à partir des photographies obtenues.

### Résultats:

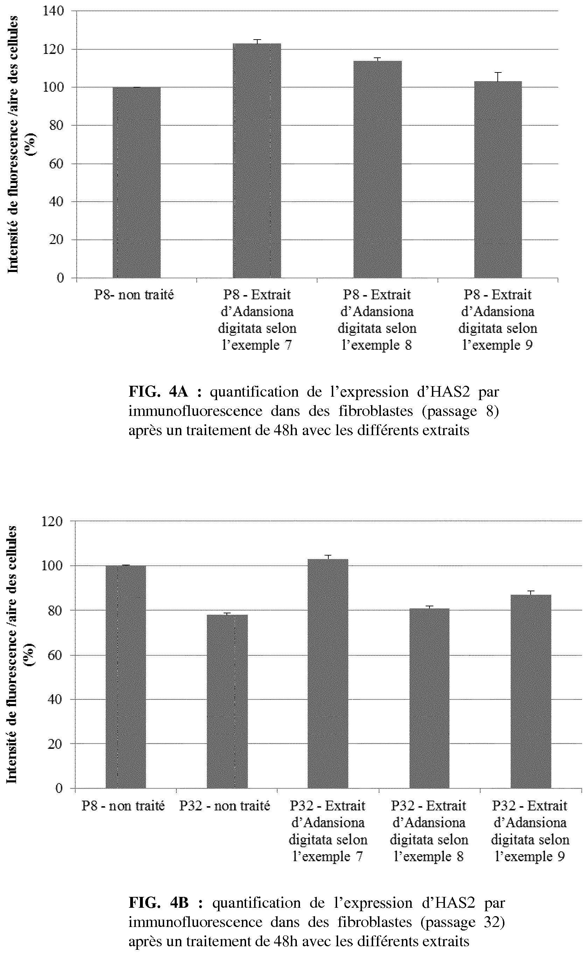

Tel qu'illustré par la Figure 4A, le traitement avec l'extrait de baobab *(Adansiona digitata)* enrichi en petits ARN d'une longueur d'au maximum 150 nucléotides, obtenu selon l'exemple 7 à 1% sur fibroblastes de passage 8 (passages considéré jeune) permet d'observer une augmentation significative de l'expression de HAS2 comparé à la condition non traitée et également par comparaison aux traitements à 1% avec les extraits *d'Adansiona digitata* obtenus selon les exemples 8 (absence de traitement par EDTA) et 9 (obtenu après précipitation).

De plus, en accord avec la littérature, tel qu'illustré par la Figure 4B, une diminution significative de l'expression de HAS2 est observée entre les fibroblastes de passage 8 (P8) et de passage 32 (P32) (sénescents).

Le traitement avec l'extrait de baobab *(Adansiona digitata)* enrichi en petits ARN selon l'invention, obtenu selon l'exemple 7 à 1% (volume/volume) sur fibroblastes sénescents permet d'observer un maintien de l'expression HAS2. Ce maintien n'est pas observé sur les cellules traitées avec les extraits *d'Adansiona digitata* obtenus respectivement selon les exemples 8 (absence de traitement par EDTA) et 9 (obtenu après précipitation).

### Conclusion:

L'extrait de baobab *(Adansiona digitata)* enrichi en petits ARN d'une longueur d'au maximum 150 nucléotides (exemple 7) à 1% stimule davantage l'expression de l'HAS2 dans les fibroblastes en comparaison à la condition non traitée et en comparaison avec les extraits *d'Adansiona digitata* obtenus selon les exemples 8 (absence de traitement par EDTA) et 9 (obtenu après précipitation).

De plus, sur fibroblastes sénescents, l'extrait de baobab *(Adansiona digitata)* enrichi en petits ARN selon l'invention (exemple 7) à 1% permet de maintenir le niveau d'expression de HAS2 observé sur les fibroblastes non sénescents. Les autres extraits *d'Adansiona digitata* non enrichis en petits ARN (exemples 8 et 9) à 1% ne permettent pas ce maintien.

### Exemple 25 : Evaluation des effets de l'extrait de gombo (Hibiscus esculentus) selon l'exemple 5 et de l'extrait de baobab (Adansiona digitata) selon l'exemple 7 sur le niveau d'expression de l'acide hyaluronique

Le but de cette étude est de visualiser l'effet des extraits de gombo *(Hibiscus esculentus)* et de baobab *(Adansiona digitata)* obtenus respectivement selon les exemples 5 et 7, extraits enrichis en ARN de petit poids moléculaire d'une longueur d'au maximum 150 nucléotides, sur l'expression de l'acide hyaluronique par marquage.

L'acide hyaluronique est un composant majoritaire de la matrice extracellulaire du derme et impliqué dans l'hydratation de la peau.

### Protocole:

Des biopsies de peau humaine de 6 mm de diamètre sont maintenues en culture *ex vivo* en présence d'un milieu spécifique (DMEM 1 g/L, HAMF12, SVF et antibiotiques) sur des inserts déposés dans des plaques 6 puits. Les biopsies sont cultivées pendant 48h et reçoivent 2 applications par jour de l'extrait de gombo *(Hibiscus esculentus)* ou de l'extrait de baobab *(Adansiona digitata*) selon les exemples 5 et 7 (respectivement) dilués à 1% (volume/volume) dans du PBS ou de PBS 1X pour la condition contrôle. Les applications sont réalisées sous forme d'une goutte d'environ 20 µl déposée à la surface de la biopsie. Les biopsies sont ensuite fixées dans le formaldéhyde puis incluses dans la paraffine. Des coupes de peau de 4 µm d'épaisseur sont ensuite réalisées. Les coupes sont incubées en présence d'une protéine biotinylée spécifique de l'acide hyaluronique (Coger, réf : 400-763-1A), puis en présence de streptavidine couplée à un fluorochrome (Invitrogen, Réf : S32354). Les biopsies sont alors examinées au microscope à Epi-fluorescence (Zeiss Axiovert 200M microscope). Une quantification de la fluorescence, à l'aide du logiciel Volocity® image analysis software (PerkinElmer, Inc.) a été réalisée à partir des photographies obtenues.

### Résultats:

Le traitement avec l'extrait de gombo *(Hibiscus esculentus)* ou l'extrait de baobab *(Adansiona digitata)* enrichi en petits ARN, obtenus respectivement selon les exemples 5 et 7, à 1% permet d'observer une augmentation significative de l'expression de l'acide hyaluronique dans l'épiderme et dans le derme comparé à la condition contrôle traitée avec du PBS 1X.

| Expression de l'acide hyaluronique | Dans l'épiderme (%) | Dans le derme (%) |
|---|---|---|
| Non traité | 100 | 100 |
| Extrait *d'Hibiscus esculentus* selon l'exemple 5 à 1% | 156 | 127 |
| Extrait *d'Adansiona digitata* selon l'exemple 7 à 1% | 159 | 124 |

### Conclusion:

L'extrait de gombo *(Hibiscus esculentus)* et l'extrait de baobab *(Adansiona digitata)* enrichi en petits ARN d'une longueur d'au maximum 150 nucléotides (selon les exemples 5 et 7) à 1% stimulent l'expression de l'acide hyaluronique dans la peau ex *vivo* humaine en comparaison à la condition contrôle traitée avec du PBS 1X.

Bien entendu, l'invention ne se limite pas aux modes de réalisation et aux exemples présentés ci-dessus et l'homme du métier, grâce à des opérations de routine, pourra être amené à réaliser d'autres modes de réalisation non décrits explicitement, qui entrent dans le cadre large de l'invention.

## Revendications

1. Procédé d'obtention d'un extrait aqueux enrichi en petits ARN d'une longueur d'au maximum 150 nucléotides à partir d'une matière végétale comprenant les étapes suivantes :
a) on met en présence la matière végétale, préalablement broyée, avec de l'eau ;
b) on ajoute de l'acide éthylène diamine tétraacétique (EDTA) tétrasodique à une concentration comprise entre 2 et 15 mM, dans le mélange obtenu en a) à un pH compris entre 10,5 et 11, cette étape étant réalisée sous agitation pendant un temps d'au moins 1h et à une température comprise entre 20 et 80°C ;
c) on ajuste ensuite le pH du mélange obtenu en b) à une valeur comprise entre 6 et 8 ;
d) on purifie le mélange obtenu en c) de manière à éliminer la matière végétale solide résiduelle et obtenir un extrait brut aqueux purifié ;
e) on contrôle le pH et on le réajuste si nécessaire à une valeur comprise entre 6 et 8, et on obtient un extrait aqueux de matière végétale, enrichi en petits ARN d'une longueur d'au maximum 150 nucléotides, comprenant en poids du poids total de l'extrait, 5-60 g/kg d'extrait sec et 10-1000 mg/kg de petits ARN d'une longueur d'au maximum 150 nucléotides, de 0,5 à 30 g/kg de fragments protéiques et de 0,5 à 50 g/kg de sucres et ne comprenant pas d'ADN,
dans lequel l'étape e) est précédée par au moins une filtration de l'extrait brut aqueux obtenu en d) et préférentiellement par des filtrations successives de l'extrait brut aqueux en abaissant le seuil de filtration de 20-50 µm à 0,1-0,30 µm.

2. Procédé selon la revendication 1 **caractérisé en ce que** dans l'étape a) on met en présence la matière végétale avec de l'eau dans un rapport matière végétale / eau de 4 à 20% (poids/poids).

3. Procédé selon la revendication 1 ou 2 **caractérisé en ce que** dans l'étape e) on contrôle le pH et on le réajuste si nécessaire à une valeur comprise entre 6 et 6,5.

4. Procédé selon l'une des revendications 1 à 3 **caractérisé en ce qu'**à l'étape d) on centrifuge le mélange obtenu en c).

5. Procédé selon l'une des revendications 1 à 4, **caractérisé en ce qu'**il comprend une étape additionnelle d'hydrolyse réalisée avant l'étape c), soit directement sur le mélange obtenu en b), soit sur le surnageant après centrifugation du mélange obtenu en b) et élimination de la matière végétale résiduelle, par action d'au moins une enzyme choisie parmi une carbohydrase, une cellulase et/ou une protéase, pendant au moins 1h, à une température comprise entre 45 et 65°C et à un pH compris entre 6 et 8,5.

6. Procédé selon la revendication 5, **caractérisé en ce qu'**il comprend une étape de désactivation de l'enzyme à une température comprise entre 65 et 80°C pendant au moins 1h, cette étape de désactivation étant réalisée directement après l'étape d'hydrolyse ou entre 2 des étapes de filtrations successives précédant l'étape e) .

7. Procédé selon l'une des revendications précédentes, **caractérisé en ce que** de la terre de diatomée ou de la silice à une concentration de 10 à 20 g/kg sont ajoutées dans le mélange avant l'étape d).

8. Procédé selon l'une des revendications précédentes, **caractérisé en ce que** la concentration en EDTA tétrasodique est de 10 mM.

9. Procédé selon l'une des revendications précédentes, **caractérisé en ce que** la matière végétale est une partie de plante choisie parmi les graines, les fruits, les bulbes, les fleurs, les feuilles, les racines, le germe ou encore les tourteaux et les drêches.

10. Procédé selon l'une des revendications précédentes, **caractérisé en ce que** la matière végétale est choisie parmi les familles des *Passifloraceae, Malvaceae, Punicaceae, Actinidiaceae, Myrtaceae, Clusiaceae, Caricaceae, Malphigiaceae, Ericaceae, Grossulariaceae, Solanaceae, Poaceae, Fabaceae, Malvaceae, Bombacaceae, Cucurbitaceae, Chenopodiaceae, Rosaceae, Rutaceae, Liliaceae, Iridaceae, Amaryllidaceae, Alliaceae.*

11. Procédé selon la revendication 10, **caractérisé en ce que** la matière végétale est choisie parmi les espèces *Hibiscus esculentus* (gombo), *Adansonia digitata* (baobab), *Chenopodium quinoa* (quinoa), *Lens esculenta* (lentille), *Oryza sativa* (germe de riz), *Cucurbita pepo* (courge), *Rosa centifolia (rose), Citrus aurantium (oranger amère), Lilium candidum (lis), Lilium tigrinum (lis tigré), Passiflora alata (grenadille).*

12. Extrait aqueux de matière végétale, enrichi en petits ARN d'une longueur d'au maximum 150 nucléotides obtenu par le procédé selon l'une des revendications 1 à 11, **caractérisé en ce qu'**il comprend en poids du poids total de l'extrait, 5-60 g/kg d'extrait sec et 10-1000 mg/kg de petits ARN d'une longueur d'au maximum 150 nucléotides, de 0,5 à 30 g/kg de fragments protéiques et de 0,5 à 50 g/kg de sucres et ne comprend pas d'ADN.

13. Extrait selon la revendication 12, **caractérisé en ce qu'**il est dilué dans un solvant et comprend en poids du poids total de l'extrait, 5-35 g/kg d'extrait sec, 0,5-20 g/kg de fragments protéiques, 0,5-30 g/kg de sucres, et 10-500 mg/kg de petits ARN d'une longueur d'au maximum 150 nucléotides.

14. Composition comprenant, en tant qu'agent actif anti-âge, une quantité efficace de l'extrait de l'une des revendications 12 ou 13, et un milieu physiologiquement acceptable.

15. Composition selon la revendication 14, **caractérisée en ce que** l'extrait est présent à une concentration comprise entre 1 et 5% en poids du poids total de la composition.

16. Composition selon l'une des revendications 14 ou 15, **caractérisée en ce qu'**elle est formulée pour être appliquée topiquement sur la peau.

17. Utilisation cosmétique de la composition de l'une des revendications 14 à 16 pour lutter contre les signes du vieillissement cutané.

18. Utilisation cosmétique de la composition de l'une des revendications 14 à 16 pour améliorer l'hydratation de la peau.

## Patentansprüche

1. Verfahren zum Erhalten eines wässrigen Extrakts, der mit kurzen RNAs mit einer Länge von maximal 150 Nucleotiden angereichert ist, aus einem pflanzlichen Material, umfassend die folgenden Schritte:
a) das zuvor zerkleinerte pflanzliche Material wird mit Wasser in Berührung gebracht;
b) Tetranatrium-Ethylendiamintetraessigsäure (EDTA) wird in einer Konzentration zwischen 2 und 15 mM der in a) erhaltenen Mischung bei einem pH zwischen 10,5 und 11 zugesetzt, wobei dieser Schritt unter Rühren während einer Zeit von mindestens 1 h und bei einer Temperatur zwischen 20 und 80 °C durchgeführt wird;
c) anschließend wird der pH der in b) erhaltenen Mischung auf einen Wert zwischen 6 und 8 eingestellt;
d) die in c) erhaltene Mischung wird gereinigt, um das restliche feste pflanzliche Material zu entfernen und einen gereinigten wässrigen Rohextrakt zu erhalten;
e) der pH wird gesteuert und er wird, wenn notwendig, erneut auf einen Wert zwischen 6 und 8 eingestellt, und es wird ein wässriger Extrakt aus pflanzlichem Material erhalten, der mit kurzen RNAs mit einer Länge von maximal 150 Nucleotiden angereichert ist, umfassend, bezogen auf das Gewicht, bezogen auf das Gesamtgewicht des Extrakts, 5 bis 60 g/kg Trockenextrakt und 10 bis 1000 mg/kg kurze RNAs mit einer Länge von maximal 150 Nucleotiden, von 0,5 bis 30 g/kg Proteinfragmente und von 0,5 bis 50 g/kg Zucker und nicht umfassend DNA,
wobei dem Schritt e) mindestens eine Filtration des in d) erhaltenen wässrigen Rohextrakts und vorzugsweise aufeinanderfolgende Filtrationen des wässrigen Rohextrakts vorausgehen, indem die Filtrationsschwelle von 20 bis 50 µm auf 0,1 bis 0,30 µm gesenkt wird.

2. Verfahren nach Anspruch 1, **dadurch gekennzeichnet, dass** in dem Schritt a) das pflanzliche Material mit Wasser in einem Verhältnis von pflanzlichem Material / Wasser von 4 bis 20 % (Gewicht/Gewicht) in Berührung gebracht wird.

3. Verfahren nach Anspruch 1 oder 2, **dadurch gekennzeichnet, dass** in dem Schritt e) der pH gesteuert wird und er, wenn notwendig, erneut auf einen Wert zwischen 6 und 6,5 eingestellt wird.

4. Verfahren nach einem der Ansprüche 1 bis 3, **dadurch gekennzeichnet, dass** in dem Schritt d) die in c) erhaltene Mischung zentrifugiert wird.

5. Verfahren nach einem der Ansprüche 1 bis 4, **dadurch gekennzeichnet, dass** es einen zusätzlichen Schritt der Hydrolyse umfasst, der vor dem Schritt c) durchgeführt wird, entweder direkt an der in b) erhaltenen Mischung oder an dem Überstand nach dem Zentrifugieren der in b) erhaltenen Mischung und Entfernen des restlichen pflanzlichen Materials, durch die Einwirkung mindestens eines Enzyms, ausgewählt aus einer Carbohydrase, einer Cellulase und/oder einer Protease, während mindestens 1 h bei einer Temperatur zwischen 45 und 65 °C und bei einem pH zwischen 6 und 8,5.

6. Verfahren nach Anspruch 5, **dadurch gekennzeichnet, dass** es einen Schritt der Deaktivierung des Enzyms bei einer Temperatur zwischen 65 und 80 °C während mindestens 1 h umfasst, wobei dieser Schritt der Deaktivierung direkt nach dem Schritt der Hydrolyse oder zwischen 2 Schritten von aufeinanderfolgenden Filtrationen, die dem Schritt e) vorausgehen, durchgeführt wird.

7. Verfahren nach einem der vorhergehenden Ansprüche, **dadurch gekennzeichnet, dass** Diatomeenerde oder Kieselerde mit einer Konzentration von 10 bis 20 g/kg der Mischung vor dem Schritt d) zugesetzt wird.

8. Verfahren nach einem der vorhergehenden Ansprüche, **dadurch gekennzeichnet, dass** die Konzentration von Tetranatrium-EDTA 10 mM beträgt.

9. Verfahren nach einem der vorhergehenden Ansprüche, **dadurch gekennzeichnet, dass** das pflanzliche Material ein Pflanzenteil ist, ausgewählt aus Samen, Früchten, Zwiebeln, Blüten, Blättern, Wurzeln, Keimen oder auch Kuchen und Treber.

10. Verfahren nach einem der vorhergehenden Ansprüche, **dadurch gekennzeichnet, dass** das pflanzliche Material ausgewählt wird aus den Familien von Passifloraceae, Malvaceae, Punicaceae, Actinidiaceae, Myrtaceae, Clusicaeae, Caricaceae, Malphigiaceae, Ericaceae, Grossulariceae, Solanaceae, Poaceae, Fabaceae, Malvaceae, Bombacaceae, Cucurbitaceae, Chenopodiaceae, Rosaceae, Rutaceae, Liliaceae, Iridaceae, Amaryllidaceae, Alliaceae.

11. Verfahren nach Anspruch 10, **dadurch gekennzeichnet, dass** das pflanzliche Material ausgewählt wird aus den Arten Hibiscus esculentus (Okra), Adansonia digitata (Baobab), Chenopodium quinoa (Quinoa), Lens esculenta (Linse), Oryza sativa (Reiskeim), Cucurbita pepo (Kürbis), Rosa centifolia (Rose), Citrus aurantium (Bitterorange), Lilium candidum (Lilie), Lilium tigrinum (Tigerlilie), Passiflora alata (Grenadille).

12. Wässriger Extrakt aus pflanzlichen Material, welcher mit kurzen RNAs mit einer Länge von maximal 150 Nucleotiden angereichert ist und welcher durch das Verfahren nach einem der Ansprüche 1 bis 11 erhalten wird, **dadurch gekennzeichnet, dass** er, bezogen auf das Gewicht, bezogen auf das Gesamtgewicht des Extrakts, 5 bis 60 g/kg Trockenextrakt und 10 bis 1000 mg/kg kurze RNAs mit einer Länge von maximal 150 Nucleotiden, von 0,5 bis 30 g/kg Proteinfragmente und von 0,5 bis 50 g/kg Zucker umfasst und keine DNA umfasst.

13. Extrakt nach Anspruch 12, **dadurch gekennzeichnet, dass** er in einem Lösungsmittel verdünnt ist und, bezogen auf das Gewicht, bezogen auf das Gesamtgewicht des Extrakts, 5 bis 35 g/kg Trockenextrakt, 0,5 bis 20 g/kg Proteinfragmente, 0,5 bis 30 g/kg Zucker und 10 bis 500 mg/kg kurze RNAs mit einer Länge von maximal 150 Nucleotiden umfasst.

14. Zusammensetzung, umfassend, als aktives Anti-Alterungsmittel, eine wirksame Menge des Extrakts nach einem der Ansprüche 12 oder 13 und ein physiologisch annehmbares Medium.

15. Zusammensetzung nach Anspruch 14, **dadurch gekennzeichnet, dass** der Extrakt in einer Konzentration zwischen 1 und 5 Gew.-%, bezogen auf das Gesamtgewicht der Zusammensetzung, vorhanden ist.

16. Zusammensetzung nach einem der Ansprüche 14 oder 15, **dadurch gekennzeichnet, dass** sie formuliert ist, um topisch auf die Haut aufgebracht zu werden.

17. Kosmetische Verwendung der Zusammensetzung nach einem der Ansprüche 14 bis 16, zur Bekämpfung der Anzeichen einer Hautalterung.

18. Kosmetische Verwendung der Zusammensetzung nach einem der Ansprüche 14 bis 16, zur Verbesserung der Hydratisierung der Haut.

## Claims

1. A method for obtaining an aqueous extract enriched with small RNAs having a maximum length of 150 nucleotides from a plant material, said method comprising the following steps:
a) bringing the plant material, crushed beforehand, into contact with water;
b) adding tetrasodium ethylenediaminetetraacetic acid (EDTA) at a concentration between 2 and 15 mM to the mixture obtained in a) at a pH between 10.5 and 11, this step being performed under stirring for a time of at least 1h and at a temperature between 20 and 80°C;
c) then adjusting the pH of the mixture obtained in b) to a value between 6 and 8;
d) purifying the mixture obtained in c) so as to remove the residual solid plant material and obtain a purified crude aqueous extract;
e) checking the pH and readjusting it if necessary to a value between 6 and 8, and obtaining an aqueous plant material extract, enriched with small RNAs having a maximum length of 150 nucleotides, comprising by weight, relative to the total weight of the extract, 5-60 g/kg of dry extract and 10-1000 mg/kg of small RNAs having a maximum length of 150 nucleotides, 0.5 to 30 g/kg of protein fragments, and 0.5 to 50 g/kg of sugars, and not comprising DNA,
wherein step e) is preceded by at least one filtration of the crude aqueous extract obtained in d) and preferably by successive filtrations of the crude aqueous extract by lowering the filtration threshold from 20-50 µm to 0.1-0.30 µm.

2. The method according to claim 1 **characterised in that** in step a) the plant material is brought into contact with water in a plant material / water ratio of 4 to 20% (weight/weight).

3. The method according to claim 1 or 2 **characterised in that** in step e) the pH is checked and readjusted if necessary to a value between 6 and 6.5.

4. The method according to one of claims 1 to 3 **characterised in that** in step d) the mixture obtained in step c) is centrifuged.

5. The method according to one of claims 1 to 4, **characterised in that** said method comprises an additional step of hydrolysis performed prior to step c), either directly on the mixture obtained in b), or on the supernatant after centrifugation of the mixture obtained in b), and removal of the residual plant material by the action of at least one enzyme selected from a carbohydrase, a cellulase and/or a protease, for at least 1h, at a temperature between 45 and 65 °C and at a pH between 6 and 8.5.

6. The method according to claim 5, **characterised in that** it comprises a step of deactivation of the enzyme at a temperature between 65 and 80 °C for at least 1h, this deactivation step being performed directly after the hydrolysis step or between 2 of the successive filtration steps preceding step e).

7. The method according to one of the preceding claims, **characterised in that** diatomaceous earth or silica are added at a concentration of 10 to 20 g/kg to the mixture before step d).

8. The method according to one of the preceding claims, **characterised in that** the concentration of tetrasodium EDTA is 10 mM.

9. The method according to one of the preceding claims, **characterised in that** the plant material is a plant part selected from the seeds, the fruits, the bulbs, the flowers, the leaves, the roots, the germ or even the press cakes and the spent grains.

10. The method according to one of the preceding claims, **characterised in that** the plant material is selected from the families of *Passifloraceae, Malvaceae, Punicaceae, Actinidiaceae, Myrtaceae, Clusiaceae, Caricaceae, Malphigiaceae, Ericaceae, Grossulariaceae, Solanaceae, Poaceae, Fabaceae, Malvaceae, Bombacaceae, Cucurbitaceae, Chenopodiaceae, Rosaceae, Rutaceae, Liliaceae, Iridaceae, Amaryllidaceae, Alliaceae.*

11. The method according to claim 10, **characterised in that** the plant material is selected from the species *Hibiscus esculentus* (okra), *Adansonia digitata* (baobab), *Chenopodium quinoa* (quinoa), *Lens esculenta* (lentil), *Oryza sativa* (rice germ), *Cucurbita pepo* (squash), *Rosa centifolia (rose), Citrus aurantium (bitter orange* tree), *Lilium candidum (lily), Lilium tigrinum (tiger lily), Passiflora alata (winged-stem passion flower).*

12. An aqueous extract of plant material, enriched with small RNAs having a maximum length of 150 nucleotides, obtained by the method according to one of claims 1 to 11, **characterised in that** it comprises by weight, relative to the total weight of the extract, 5-60 g/kg of dry extract and 10-1000 mg/kg of small RNAs having a maximum length of 150 nucleotides, 0.5 to 30 g/kg of protein fragments, and 0.5 to 50 g/kg of sugars, and does not comprise DNA.

13. The extract according to claim 12, **characterised in that** it is diluted in a solvent and comprises by weight, relative to the total weight of the extract, 5-35 g/kg of dry extract, 0.5-20 g/kg of protein fragments, 0.5-30 g/kg of sugars, and 10-500 mg/kg of small RNAs having a maximum length of 150 nucleotides.

14. A composition comprising, as active anti-aging agent, an effective amount of the extract according to one of claims 12 or 13 and a physiologically acceptable medium.

15. The composition according to claim 14, **characterised in that** the extract is present in a concentration between 1 and 5 % by weight relative to the total weight of the composition.

16. The composition according to one of claims 14 or 15, **characterised in that** it is formulated so as to be suitable for topical application on the skin.

17. Cosmetic use of the composition according to one of claims 14 to 16 for combatting the signs of skin aging.

18. Cosmetic use of the composition according to one of claims 14 to 16 for improving the hydration of the skin.
